# EUROPÄISCHE PATENTANMELDUNG

(11) **EP 4 837 565 A1**
(43) Veröffentlichungstag der Anmeldung: **11.11.2026**
(21) Anmeldenummer: 25174753.1
(22) Anmeldetag: 07.05.2025
(51) Int. Cl.: A61K 9/16, A61K 31/12, A61K 31/352, A61K 31/357, A61K 31/4375, A61K 36/738, A61K 47/69

(54) **ZUSAMMENSETZUNG MIT BESCHICHTETEN CYCLODEXTRIN-KOMPLEXEN**

(71) Anmelder: Evanium Healthcare GmbH, 93053 Regensburg (DE)
(72) Erfinder: OFNER, David, 93047 Regensburg (DE); ROLKA, Felix, 93047 Regensburg (DE)
(74) Vertreter: Weickmann & Weickmann PartmbB

(57) **Zusammenfassung**

Zusammensetzung, umfassend mindestens einen Cyclodextrin-Komplex, worin mindestens ein Wirkstoff mit gamma-Cyclodextrin assoziiert ist, wobei der Cyclodextrin-Komplex eine Beschichtung aufweist, die Hydroxypropylstärke und Phosphatidylcholin umfasst, wobei der Gewichtsanteil des Wirkstoffs 5-50 Gew.-% beträgt, bevorzugt 10-40 Gew.-%, der Gewichtsanteil des gamma-Cyclodextrins 35-90 Gew.-% beträgt, bevorzugt 50-85 Gew.-%, der Gewichtsanteil der Hydroxypropylstärke 0,1-10 Gew.-% beträgt, bevorzugt 0,5-7,55 Gew.-%, weiter bevorzugt 0,75-4 Gew.-%, besonders bevorzugt 1-3,5 Gew.-%, und der Gewichtsanteil des Phosphatidylcholins 0,1-10 Gew.-% beträgt, bevorzugt 0,5-7,5 Gew.-% beträgt, weiter bevorzugt 1-4,5 Gew.-%, besonders bevorzugt 1,5-4 Gew.-%, jeweils bezogen auf die Gesamtmasse des beschichteten Komplexes.

## Beschreibung

Die Erfindung betrifft ein System aus beschichteten Cyclodextrin-Komplexen, die mit einem Wirkstoff beladen sind, der in gamma-Cyclodextrin eingeschlossen ist. Außerdem wird deren Verwendung zur Verbesserung der Bioverfügbarkeit bei oraler Verabreichung sowie ein Verfahren zur Herstellung der beschichteten Cyclodextrin-Komplexe beschrieben.

### Hintergrund

Wirkstoffe mit niedriger Wasserlöslichkeit führen bei oraler Aufnahme oftmals auch zu einer niedrigen Bioverfügbarkeit. Die geringe Löslichkeit führt hierbei zu einer unvollständigen Freisetzung während des Verdauungsprozesses und infolgedessen auch geringerer Aufnahme.

Um das Problem der geringen Löslichkeit zu überwinden, wurden im Fachbereich unterschiedliche Techniken entwickelt. In diesem Zusammenhang wurde die Komplexierung von Wirkstoffen mit Cyclodextrinen, ringförmigen Stärkemolekülen beschrieben. Hierbei verdrängt der Wirkstoff Wasser aus dem Hohlraum des Cyclodextrins und bildet einen Einschlusskomplex, stabilisiert durch nicht-kovalente Bindungen zwischen den Glucosemolekülen des Cyclodextrins und dem schwerlöslichen Wirkstoff. Gängige Cyclodextrine sind hierbei alpha-Cyclodextrin (6 Glucosemoleküle), beta-Cyclodextrin (7 Glucosemoleküle) und gamma-Cyclodextrin (8 Glucosemoleküle). In der Literatur sind verschiedene Methoden zur Komplexbildung beschrieben, wobei Wirkstoff und Cyclodextrin unter Rühren - teilweise unter Einwirkung von Scherkräften, Temperaturerhöhung oder pH Schwankungen - den Einschlusskomplex ausbilden. Diese Reaktion wird durch die Komplexbildungskonstante Ks beschrieben, welche insbesondere von der Struktur des Wirkstoffes, dessen Polarität, Löslichkeit und Interaktion mit dem Cyclodextrin abhängig ist. Gängige Herstellverfahren zielen nun auf eine Erhöhung der Ausbeute bei gleichzeitiger Zeitersparnis ab, wobei der finale Cyclodextrin-Komplex in seinen Eigenschaften grundsätzlich ähnlich bis identisch ist.

In der Literatur ist ebenfalls beschrieben, dass Komplexe mit nativen Cyclodextrinen und insbesondere Komplexe mit gamma-Cyclodextrin trotz teilweise hoher Komplexbildungskonstanten und vorteilhafter Komplexgeometrie nur geringe Löslichkeit besitzen. Dies liegt an der Ausbildung von supramolekularen Aggregaten, welche ein Kristallgitter ausbilden können und "Stacking" betrieben. Dies führt zu geringer Löslichkeit und schlechtem Dissolutionsverhalten von gamma-Cyclodextrin Komplexen selbst bei Verwendung von Herstellmethoden mit hoher Ausbeute und effektiver Komplexbildung. Aus diesem Grund wurde gamma-Cyclodextrin für den Zweck der Löslichkeitsverbesserung im Fachbereich bisher nur seltener eingesetzt als andere Cyclodextrine.

Die gängigen Methoden der Komplexbildung mit Cyclodextrinen führen häufig nicht zu einer ausreichenden Verbesserung der Bioverfügbarkeit oral verabreichter Wirkstoffe. Die Aufgabe der Erfindung war es daher, eine Möglichkeit zur weiteren Verbesserung der Bioverfügbarkeit schwerlöslicher Wirkstoffe zu entwickeln.

### Beschreibung

Überraschenderweise wurde nun gefunden, dass sich durch die Kombination von vorzugsweise niedrigviskoser Hydroxypropylstärke mit Phospholipiden in einem bestimmten Verhältnis und Einsatzkonzentration sowie spezieller Verfahren gamma-Cyclodextrin-komplexe mit signifikant höherer Löslichkeit und Dissolution im Vergleich zu nativen Komplexen herstellen lassen.

Ein erster Aspekt der Erfindung betrifft daher eine Zusammensetzung, die einen mit gamma-Cyclodextrin komplexierten Wirkstoff umfasst, wobei der Komplex eine Beschichtung aufweist, die Hydroxypropylstärke und Phosphatidylcholin umfasst.

Eine erfindungsgemäße Zusammensetzung umfasst mindestens einen Cyclodextrin-Komplex, worin mindestens ein Wirkstoff mit gamma-Cyclodextrin assoziiert ist, wobei der Cyclodextrin-Komplex eine Beschichtung aufweist, die Hydroxypropylstärke und Phosphatidylcholin umfasst,
wobei der Gewichtsanteil des Wirkstoffs 5-50 Gew.-% beträgt, bevorzugt 10-40 Gew.-%,
der Gewichtsanteil des gamma-Cyclodextrins 35-90 Gew.-% beträgt, bevorzugt 50-85 Gew.-%,
der Gewichtsanteil der Hydroxypropylstärke 0,1-10 Gew.-% beträgt, bevorzugt 0,5-7,5 Gew.-%, weiter bevorzugt 0,75-4 Gew.-%, besonders bevorzugt 1-3,5 Gew.-%, und der Gewichtsanteil des Phosphatidylcholins 0,1-10 Gew.-% beträgt, bevorzugt 0,5-7,5 Gew.-% beträgt, weiter bevorzugt 1-4,5 Gew.-%, besonders bevorzugt 1,5-4 Gew.-%,
jeweils bezogen auf die Gesamtmasse des beschichteten Komplexes.

Die Zusammensetzung liegt vorzugsweise in fester Form vor, da die beschichteten Cyclodextrin-Komplexe dann eine besonders gute Stabilität aufweisen. Alternativ kann die Zusammensetzung auch in wässriger Lösung vorliegen, beispielsweise kann sie direkt vor einer gewünschten Verwendung in Lösung gebracht werden.

Als schwerlösliche Wirkstoffe werden im Sinne der Erfindung Wirkstoffe mit einer Löslichkeit bis 10mg/ml in reinem Wasser bei 20°C angesehen:

| **Löslichkeitsklassifikation** | **Absolute Löslichkeit (dest. Wasser, 20°C)** |
|---|---|
| Very Soluble | >1000mg/ml |
| Freely Soluble | 100-1000mg/ml |
| Soluble | 33-100mg/ml |
| Sparingly Soluble | 10-33mg/ml |
| Slightly Soluble | 1-10mg/ml |
| Very Slightly Soluble | 0,1-1mg/ml |
| Practically Insoluble | <0,1mg/ml |

Für entsprechende Wirkstoffe in der Kategorie "Practically Insoluble" sowie "Very Slightly Soluble" sowie im weiteren Sinne auch bis "Slightly Soluble", ist die effektive Gabe über orale Darreichungen problematisch.

Überraschenderweise wurde gefunden, dass entsprechende Wirkstoffe sehr effektiv in Lösung gehen bzw. freigesetzt werden, wenn Sie in Form eines gamma-Cyclodextrin Komplexes formuliert werden, welcher zusätzlich mit Hydroxypropylstärke und Phospholipiden gecoated wird.

Sowohl Phospholipide, modifizierte Stärke, als auch Cyclodextrine werden als häufig zur Löslichkeitserhöhung eingesetzt. Während Phospholipide häufig zur Ausbildung von liposomalen Strukturen genutzt werden, agieren modifizierte Stärken als Mizellenbildner und Cyclodextrine als Komplexbildner. Die Mechanismen der Löslichkeitserhöhung sind jedoch im besten Fall additiv, in den meisten Fällen eher antagonistisch, da in der Praxis der Wirkstoff mit den Phospholipiden bzw. den funktionellen Gruppen der modifizierten Stärke um die Komplexbildung konkurriert. Überraschenderweise wurde jedoch gefunden, dass ein bestimmtes Konzentrationsverhältnis in Kombination mit einem abschließenden Coatingverfahren eine deutlich überadditive Löslichkeit ermöglicht.

Zentraler Bestandteil ist hierbei neben der Auswahl von gamma-Cyclodextrin das abschließende Coating, für welches Hydroxypropyl-modifizierte Stärke sowie Phosphatidylcholin genutzt werden.

Der Gewichtsanteil des gamma-Cyclodextrins beträgt 35-90 Gew.-%, bezogen auf die Gesamtmasse des beschichteten Komplexes. Besonders vorteilhaft ist ein Gehalt im Bereich von 50-85 Gew.-% Gamma-Cyclodextrin. Für andere Cyclodextrintypen wie alpha- oder beta-Cyclodextrin kann keine überadditive Steigerung der Löslichkeit durch das erfinderische Verfahren bzw. die erfinderische Zusammensetzung erzielt werden.

Phosphatidylcholin, ggf. in Kombination mit weiteren Phospholipiden, dient erfindungsgemäß zusammen mit Hydroxypropylstärke dem Coating des gamma-Cyclodextrinkomplexes sowie der Interaktion mit gamma-Cyclodextrin, wodurch die Löslichkeit signifikant gesteigert werden kann.

Unter Phospholipiden versteht man eine Gruppe von lipidischen Molekülen, die eine zentrale Rolle in biologischen Membranen spielen. Sie bestehen aus Fettsäuren, einem Glycerinmolekül, einer Phosphatgruppe und einem polarisierten Kopf, der oft mit einer zusätzlichen chemischen Gruppe (z. B. Cholin, Ethanolamin, Serin) verbunden ist. Im Sinne der Erfindung können aufgereinigte Phospholipide mit einem Phosphatidylcholin-Anteil von mindestens 40%, bevorzugt > 70%, besonders bevorzugt > 80%, am besten > 90%, genutzt werden. Die Menge der Phospholipide wird so gewählt, dass der Anteil an Phosphatidylcholin (PC) in der Komplexbeschichtung, bezogen auf die Gesamtmasse des beschichteten Komplexes, etwa 0,1-10 Gew.-% beträgt, bevorzugt 0,5-7,5 Gew.-%. Besonders vorteilhaft sind Anteile im Bereich von 1-4,5 Gew.-%, vorzugsweise 1,5-4 Gew.-% Phosphatidylcholin bezogen auf die Gesamtmasse des beschichteten Komplexes.

Hoch aufgereinigte Phospholipide und insbesondere das im Sinne der Erfindung relevante Phosphatidylcholin werden hauptsächlich aus pflanzlichen oder tierischen Quellen, insbesondere aus Sojabohnen, Sonnenblumenkernen oder Eidotter hergestellt. Der Prozess umfasst mehrere Schritte, darunter Extraktion, Reinigung und Fraktionierung, um den gewünschten Reinheitsgrad zu erreichen. Zunächst werden die Ausgangsrohstoffe mittels eines unpolaren Lösemittels wie Hexan extrahiert oder die fettreiche Fraktion mittels mechanischer Pressung gewonnen und anschließend raffiniert. Hiernach wird durch Entölung (z.B. durch Wasserhydration oder Acetonbehandlung) das Rohlecithin gewonnen, welches eine Mischung verschiedener Phospholipide, insbesondere Phosphatidylcholin (PC), Phosphatidylserin (PS), Phosphatidylethanolamin (PE), Phosphatidylinositol (PI) und Sphinomyelin. Durch Fraktionierung bzw. Aufreinigung werden die Bestandteile aufgereinigt, dies erfolgt entweder durch enzymatische Spaltung ungewünschter Phospholipide durch Phospholipasen oder/und durch chromatographische Trennung durch Adsorptionschromatographie mit einem geeigneten Lösemittel wie Ethanol oder Aceton. Auch eine Extraktion mittels überkritischem CO₂ ist möglich.

Relevant zur Klassifizierung eines Phospholipids ist insbesondere:
1. Der Anteil an Phosphatidylcholin (PC) bezogen auf die Gesamtphospholipide und angegeben in Gew.-%
2. Der Anteil anderer Phospholipide, insbesondere PS, PE, PI bezogen auf die Gesamtphospholipide und angegeben in Gew.-%

Im Sinne der Erfindung genutzt werden vorzugsweise reines Phosphatidylcholin oder aufgereinigte Phospholipide mit einem PC-Anteil von mindestens 40%, bevorzugt >70%, besonders bevorzugt >85%, am besten >90%. Neben PC sind auch andere Phospholipide in der Lage, in die erfindungsgemäße Beschichtung eines Cyclodextrin-Komplexes aufgenommen zu werden. Daher kann die Beschichtung zusätzlich zu PC auch ein oder mehrere weitere Phospholipide umfassen.

Modifizierte Stärke wird ausschließlich in Form von Hydroxpropylstärke eingesetzt. Unter Hydroxypropylstärke werden erfindungsgemäß insbesondere Stärkeether-Derivate verstanden, bei denen Hydroxygruppen der Glucose-Einheit der Stärke mit Propylenoxid verethert sind. Der Gewichtsanteil der Hydroxypropylstärke in der Komplexbeschichtung beträgt bezogen auf die Gesamtmasse des beschichteten Komplexes 0,1-10 Gew.-%, bevorzugt 0,5-7,5 Gew.-%. Besonders vorteilhaft sind Anteile im Bereich von 0,75-4 Gew.-%, vorzugsweise 1-3,5 Gew.-% Hydroxypropylstärke bezogen auf die Gesamtmasse des beschichteten Komplexes.

Wesentlich bei der Auswahl einer geeigneten Hydroxypropylstärke ist zum einen ihr Substitutionsgrad und zum anderen ihre Viskosität. Der Substitutionsgrad, angegeben in Gewichtsprozent der modifizierten Einheit, bezieht sich auf die Trockenmasse, und kann durch Kernspinresonanzspektroskopie (z.B. beschrieben in Ph. Eur. 11.0 Gehaltsbestimmung Amylum hydroxypropylum pregeleficatum) ermittelt werden. Erfindungsgemäß verwendete Stärke weist vorzugsweise einen Substitutionsgrad von 0,1-15 %, bevorzugt 0,5-10 %, besonders bevorzugt 2-7 % auf. Die Viskosität der modifizierten Stärke kann mittels Rotationsviskosimetrie gemessen werden. Die Methode der Rotationsviskosimetrie wird in der Norm ISO 1652:2011 *Rubber latex - Determination of apparent viscosity by the Brookfield test method* erläutert. Beispielsweise kann die Messung unter Verwendung eines Brookfield LV Viskosimeter, Spindel LV1 als 10 Gew.-% Lösung bei 25°C durchgeführt werden. Im Sinne der Erfindung werden besonders bevorzugt Stärken mit einer geringen Viskosität von (< 250mPas, bevorzugt < 100mPas, besonders bevorzugt < 75mPas, am besten < 50mPas) eingesetzt, wie gemäß der Norm ISO 1652:2011 bei 25°C als 10 Gew.-% Lösung gemessen. Hydroxypropylstärken mit einer Viskosität in diesem Bereich sind besonders gut geeignet, um eine gleichmäßige Beschichtung zu erreichen, die zudem die Stabilität der Formulierung nicht beeinträchtigt. Bei höher viskosen Stärken ist die Ummantelung dagegen so stark ausgeprägt, dass es zu einer Instabilität der Formulierung kommt.

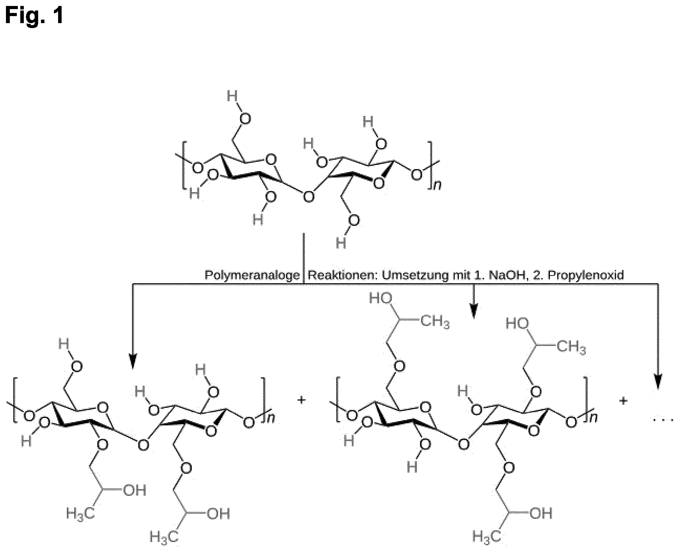

Geeignete Stärken für die erfindungsgemäße Verwendung können erhalten werden, indem Stärke nach der Gelatinisierung sowie dem Einkürzen der Kettenlängen durch enzymatische Behandlung oder Dextrinisierung (Fluidisierung) mit Propylenoxid behandelt wird, wobei die in Fig. 1 gezeigte Struktur entsteht (Hydroxypropylstärke).

Relevant zur Klassifikation der Stärken sind insbesondere
1. Substitutionsgrad, angegeben in Gewichtsprozent der modifizierten Einheit, bezogen auf die Trockenmesse, ermittelt durch NMR
2. Viskosität der Stärke, im Sinne der Erfindung gemessen mittels Rotationsviskosimetrie, zum Beispiel mit einem Brookfield LV Viskosimeter, Spindel LV1 als 10Gew.-% Lösung bei 25°C

Im Sinne der Erfindung genutzt werden besonders bevorzugt Stärken mit einer geringen Viskosität (< 250mPas, bevorzugt < 100mPas, besonders bevorzugt < 75mPas, am besten < 50mPas 10 Gew.-%w/w Lösung bei 25°C) sowie einem Substitutionsgrad von 0,1-15%, bevorzugt 0,5-10%, besonders bevorzugt 2-7% Hydroxypropylgruppen bezogen auf die Trockenmasse (gemessen gemäß der Norm ISO 1652:2011 bei 25°C als 10 Gew.-% Lösung).

Der kombinierte Gewichtsanteil von Phosphatidylcholin + Hydroxypropylstärke in der Komplexbeschichtung beträgt vorteilhafterweise 0,5-15 Gew.-%, bevorzugt 1-10 Gew.-%, weiter bevorzugt 1,5-8,5 Gew.-%, besonders bevorzugt 2-7 Gew.-%, bezogen auf die Gesamtmasse des beschichteten Komplexes. Darüber hinaus ist es möglich, dass eine erfindungsgemäße Zusammensetzung zusätzliche Mengen an Stärke und Phosphatidylcholin umfassen kann, die nicht Teil der Beschichtung des Cyclodextrin-Komplexes sind.

In einer erfindungsgemäßen Zusammensetzung liegen die beschichteten Cyclodextrin-Komplexe insbesondere in Form von Agglomeraten mehrerer einzelner Komplexe vor.

Aufgrund der erfindungsgemäßen Beschichtung lösen sich diese Agglomerate besonders leicht wieder auf (in kleinere Agglomerate oder insbesondere einzelne beschichtete Cyclodextrin-Komplexe), wenn die Zusammensetzung in Lösung gebracht oder z.B. oral verabreicht wird.

Die Partikelgröße der einzelnen beschichteten Cyclodextrinkomplex-Partikel in Lösung beträgt etwa 0,1-10 µm, bevorzugt 0,2-5 µm, besonders bevorzugt 0,3-4 µm, ausgedrückt als D50-Wert, welcher mittels Laserbeugung gemessen wird, beispielsweise mit einem Mastersizer 3000 von Malvern Panalytical.

In bevorzugten Ausführungsformen der Erfindung umfasst die Zusammensetzung weiterhin ein oder mehrere Trägerstoffe bzw. Lyoprotektoren. Dies sind erfindungsgemäß Verbindungen, die wasserlöslich und in der Lage sind, Wasserstoffbrücken mit den beschichteten Cyclodextrin-Komplexen auszubilden. Sie ersetzen beim Trocknungsvorgang die Wassermoleküle und stabilisieren die beschichteten Komplexe durch Ausbildung von Wasserstoffbrücken. Besonders bevorzugte Trägerstoffe bzw. Lyoprotektoren sind Saccharide, insbesondere in Form von Maltodextrin. Diese können aus der Gruppe ausgewählt werden, die Maltodextrin, Glucose, Saccharose, Fructose, Maltose, Lactose, Galactose, Trehalose, Mannose und Kombinationen davon umfasst. Bevorzugt wird der Einsatz von Stärkehydrolysaten mit einem Dextroseäquivalent (DE) von 3-50, besonders bevorzugt mit einem DE von 5-30, am besten von 10-20. Der Gewichtsanteil der Trägerstoffe bzw. Lyoprotektoren, insbesondere Saccharide, kann, bezogen auf die Gesamtmasse des beschichteten Komplexes, insgesamt 5-50 Gew.-% betragen, bevorzugt 10-40 Gew.-%, bevorzugt 12,5-35 Gew.-% besonders bevorzugt 15-30 Gew.%.

Wirkstoffe, die in den erfindungsgemäßen beschichteten Cyclodextrin-Komplexen verkapselt werden können, sind insbesondere hydrophobe Wirkstoffe mit einer geringen Wasserlöslichkeit von 10 mg/ml und weniger (in reinem Wasser bei 20°C). Besonders vorteilhaft ist die erfindungsgemäße Verkapselung für Wirkstoffe mit einer Wasserlöslichkeit von < 1 mg/ml. Das Molekulargewicht der Wirkstoffe kann im Bereich 150 -2000 g/mol liegen. Die chemische Struktur der Wirkstoffe ist grundsätzlich beliebig wählbar, sofern sie zu einer nicht-kovalenten Wechselwirkung mit gamma-Cyclodextrin in der Lage sind. Es kann sich um natürliche oder synthetische Stoffe mit einer geringen Wasserlöslichkeit handeln.

Bevorzugt sind vor allem schwerlösliche sekundäre Pflanzenstoffe und deren Glycoside wie Stilbenoide (Resveratrol, Pterostilben, Piceatannol, Rhaponticin, Desoxyrhaponticin, Gnetin), Flavonoide, insbesondere Flavonole (Fisetin, Quercetin, Kaempferol, Rutin, Myricetin, Isorhamnetin), Flavanone (Naringenin, Hesperitin), Flavanole (Catechin, Epicatechin, Epigallocatechin, Epicatechingallat, Epigallocatechingallat, Theaflavine), Isoflavone (Genistein, Daidzein, Glycetein), Anthocyanidine/Antocyane (Cyanidin, Delphinidin, Malvidin), Dihydroflavonole (Taxifolin, Dihydrokaempferol, Dihydromyricetin), Neoflavonoide (Pterocarpan, Coumestrol), Flavonolignane (Silibinin, Isosilybin, Silychristin, Silydianin), Flavone (Apigenin, Luteolin, Baicalein, Chrysin, Tangeretin), C-Glykosylflavone (Isovitexin, Vitexin), Monoterpene (Thujon, Limonen, Menthol, Carvon, Cineol, Pinen, Linalool), Diterpene (Ginkgolide, Carnosol, Carnosolsäure, Forskolin), Sesquiterpene (beta-Caryophyllen), Triterpene (Lupeol, Ursolsäure, Betulinsäure, beta-Boswelliasäure, alpha-Boswelliasäure, 11-keto-beta-Boswelliasäure, 3-O-acetyl-11-keto-beta-Boswelliasäure), Chalcone (Xanthohumol, Phloretin, Butein, Flavokavain, Hesperetin- bzw. Naringenin-Chalcon), Curcuminoide (Curcumin, Demethoxycurcumin, Bisdemethoxycurcumin, Tetrahydrocurcumin), Lignane (Secoisolariciresinol, Matairesinol, Enterolacton), Hydrochinonderivate (Thymochinon), Zimtsäurederivate (Ferulasäure, Cumarinsäure, Chlorogensäure, Rosmarinsäure), Gingerole ([6]-Gingerol, [8]-Gingerol, [10]-Gingerol), Shogaole ([6]-Shogaol, [8]-Shogaol, [10]-Shogaol), (Steroide (beta-Sitosterol, Campesterol, Stigmasterol, Gamma-Oryzanol), Xanthone (Mangostin, Norathyriol) und Anthraquinone (Emodin, Aloe-Emodin, Rhein), Carotinoide (β-Carotin, Lutein, Zeaxanthin, Lycopin, Astaxanthin), Alkaloide (Berberin, Capsaicin, Coffein, Theobromin, Piperin), Saponine (Ginsenoside), Tocopherole (alpha-, beta-, gamma-, delta-Tocopherol), Tocotrienole (alpha-, beta-, gamma-, delta-Tocotrienol), Glucosinolate (Sulforaphan).

Daneben ist die Anwendung ebenfalls bevorzugt für weitere schwerlösliche Stoffe wie Alpha-Liponsäure, Ubichinon, Ubichinol, Melatonin, Menadion, Menaquinon, Phylloquinon, Cholecalciferol, Ergocalciferol, 22-Dihydroergocalciferol, Sitocalciferol, Dehydroepiandrosteron, Glutathion, Omega-3 Fettsäuren sowie deren Ester (Alpha-Linolensäure, Eicosapentaensäure, Docosahexaensäure).

Darüber hinaus ist die Anwendung bevorzugt für schwerlösliche pharmazeutische Wirkstoffe wie Antibiotika (Amoxicillin, Ciprofloxacin, Azithromycin, Tetracyclin), Analgetika (Ibuprofen, Diclofenac, Ketoprofen, Naproxen), Antidiabetika (Metformin, Glibenclamid, Pioglitazon, Glimepirid), Antihypertensiva (Losartan, Atenolol, Hydrochlorothiazid, Verapamil), Antidepressiva (Amitriptylin, Fluoxetin, Sertralin, Paroxetin), Antiemetika (Ondansetron, Metoclopramid, Domperidon, Prochlorperazin), Antikoagulanzien (Warfarin, Dabigatran, Apixaban), Antimykotika (Itraconazol, Ketoconazol, Fluconazol, Terbinafin), Antiviral (Acyclovir, Oseltamivir, Lamivudin), Antitumor-Wirkstoffe (Paclitaxel, Doxorubicin, Docetaxel, Vincristin), Neuroleptika (Haloperidol, Olanzapin, Quetiapin, Clozapin), Sedativa und Hypnotika (Diazepam, Lorazepam, Midazolam, Clonazepam), Lipidregulatoren (Atorvastatin, Simvastatin, Lovastatin, Pravastatin), Immunosuppressiva (Cyclosporin A, Tacrolimus, Mycophenolatmofetil), Antikonvulsiva (Phenytoin, Carbamazepin, Valproinsäure, Lamotrigin), Protonenpumpenhemmer (Omeprazol, Lansoprazol, Pantoprazol), Corticosteroide (Prednison, Dexamethason, Hydrocortison), Vitamin D Analoga (Calcipotriol, Paricalcitol, Tacalcitol), Analgetische Opioide (Morphin, Oxycodon, Fentanyl, Buprenorphin), Retinoide (Isotretinoin, Tretinoin).

Der Gewichtsanteil des Wirkstoffs an der Gesamtmasse des beschichteten Komplexes liegt bei 5-50 Gew.-%. Besonders vorteilhaft sind Wirkstoffbeladungen im Bereich von 10-40 Gew.-% Wirkstoff bezogen auf die Gesamtmasse des beschichteten Komplexes.

Der kombinierte Gewichtsanteil des Wirkstoffes + gamma-Cyclodextrin an der Gesamtmasse des beschichteten Komplexes beträgt vorzugsweise zwischen 85-99 Gew.-%, bevorzugt 90-98,5 Gew.-%, besonders bevorzugt etwa 93-98 Gew.-%.

Die erfindungsgemäße Zusammensetzung ist aufgrund der verbesserten Löslichkeit und Dissolution besonders gut für die orale Darreichungen geeignet. Sie kann beispielsweise in Form einer Tablette, Kapsel, Kau-, Schmelz- oder Brausetablette, als Granulat, als Pulver, in Lösung für die orale Einnahme, als Dragee, als Sublingual/Buccaltablette sowie als Schmelzfilm vorliegen.

Neben den beschichteten Cyclodextrin-Komplexen kann eine erfindungsgemäße Zusammensetzung weitere übliche Bestandteile einer für die orale Verabreichung vorgesehenen Formulierung umfassen.

Die erfindungsgemäße Zusammensetzung kann als pharmazeutische oder nutrazeutische Zusammensetzung sowie als Lebensmittel formuliert werden. In einer bevorzugten Ausführungsform ist die erfindungsgemäße Zusammensetzung für die nutrazeutische Anwendung vorgesehen.

Abhängig von einer gewünschten Verwendung der beschichteten Cyclodextrin-Komplexe können sie zu einer Zusammensetzung für die orale Verabreichung weiterverarbeitet werden. Hierzu kann die Zusammensetzung beispielsweise als Tablette, Kapsel, Kau-, Schmelz- oder Brausetablette, als Granulat, als Pulver, als Lösung für die orale Einnahme, als Dragee, als Sublingual/Buccaltablette sowie als Schmelzfilm formuliert und gegebenenfalls mit weiteren Bestandteilen kombiniert werden.

Ein weiterer Aspekt der Erfindung betrifft ein Verfahren zur Herstellung einer oben beschriebenen Zusammensetzung, die beschichtete Cyclodextrin-Komplexe umfasst, wobei das Verfahren die folgenden Schritte umfasst:
a) Dispergieren von gamma-Cyclodextrin und mindestens einem Wirkstoff in Wasser unter Rühren, um den Wirkstoff mit dem gamma-Cyclodextrin zu assoziieren,
b) Beschichten des in Schritt (a) gebildeten Cyclodextrin-Komplexes durch Zugeben von Hydroxypropylstärke und Phosphatidylcholin unter Rühren und Homogenisation, um eine Suspension beschichteter Cyclodextrin-Komplexe in Wasser zu erhalten, und
c) ggf. Trocknen der in Schritt (b) erhaltenen Suspension.

Bei der Herstellung einer wässrigen Dispersion in Schritt a) erfolgt eine Assoziierung des Wirkstoffs mit dem gamma-Cyclodextrin. Hierzu kann eine wässrige Lösung des gamma-Cyclodextrins vorgelegt und anschließend Wirkstoff zugegeben werden. Die Dispergierung erfolgt vorzugsweise unter Rühren, beispielsweise in einem High Shear Homogenisator.

Unter einer Assoziierung wird im Sinne der Erfindung eine nicht-kovalente Wechselwirkung des Wirkstoffs mit dem gamma-Cyclodextrin verstanden, wobei der Wirkstoff ganz oder teilweise in den Hohlraum des gamma-Cyclodextrins aufgenommen werden kann, sodass sich ein Einschlusskomplex bildet, in dem der Wirkstoff in das gamma-Cyclodextrin eingelagert ist. Durch Einstellen des pH-Werts kann die Komplexierung des Wirkstoffes mit gamma-Cyclodextrin gefördert werden.

Das Gewichtsverhältnis von gamma-Cyclodextrin und Wirkstoff wird so gewählt, dass der Wirkstoff möglichst vollständig in das Cyclodextrin aufgenommen wird. Vorteilhafterweise liegt der Gewichtsanteil des Wirkstoffs bei etwa 5-50 Gew.-% und der Gewichtsanteil des gamma-Cyclodextrins bei etwa 35-90 Gew.-%, jeweils bezogen auf die Gesamtmasse des beschichteten Komplexes. Bevorzugte Gewichtsanteile sind wie vorstehend im Zusammenhang mit einer erfindungsgemäßen Zusammensetzung beschrieben.

Im anschließenden Schritt b) werden der in Schritt a) erhaltenen Dispersion Hydroxypropylstärke und Phosphatidylcholin zugegeben, und es erfolgt eine Homogenisierung. Dadurch ordnen sich Hydroxypropylstärke und Phosphatidylcholin in der wässrigen Dispersion zu einer Hülle um den Cyclodextrin-Komplex an, welche den mit gamma-Cyclodextrin assoziierten Wirkstoff umschließt. Die Homogenisierung kann mittels Hockdruck-Homogenisation, Ultraschall-Homogenisation oder Vermahlen, beispielsweise in einer Kugelmühle erfolgen.

Im Falle der Hochdruck Homogenisation erfolgt die Beschichtung zum Beispiel durch Passage bei niedrigem bis mittlerem Druck (100-800 bar), wobei typischerweise 1-5 Zyklen nötig sind. Für die Homogenisation mittels Kugelmühle werden bevorzugt Mahlkörper mit Durchmessern von 0,05-1mm, bevorzugt 0,1-0,8mm, besonders bevorzugt 0,3-0,4mm gewählt, welche aus Edelstahl, Glas, Zirkonoxid, Wolframcarbid, Aluminiumoxid, PTFE oder Achat gefertigt wurde, besonders bevorzugt sind Mahlkörper aus Zirkonoxid. Bei der Homogenisation durch Ultraschall können entweder Sonotroden, Ultraschallbäder oder Durchfluss-Ultraschallhomogenisatoren/Ultraschallkammern zum Einsatz kommen.

Die Menge des Phosphatidylcholins wird vorzugsweise so gewählt, dass sein Gewichtsanteil in der Beschichtung, bezogen auf die Gesamtmasse des beschichteten Komplexes, etwa 0,1-10 Gew.-%, bevorzugt 0,5-7,5 Gew.-% beträgt. Besonders geeignet sind Gewichtsanteile von 1-4,5 Gew.-% oder 1,5-4 Gew.-% an Phosphatidylcholin, bezogen auf die Gesamtmasse des beschichteten Komplexes. Phosphatidylcholin (PC) kann erfindungsgemäß in Form eines Gemischs von Phospholipiden eingesetzt werden. Im Sinne der Erfindung können aufgereinigte Phospholipide mit einem PC-Anteil von mindestens 40 %, bevorzugt > 70%, besonders bevorzugt > 80%, am besten > 90%, genutzt werden.

Die Menge der eingesetzten Hydroxypropylstärke wird so gewählt, dass ein Gewichtsanteil in der Beschichtung von etwa 0,1-10 Gew.-%, bevorzugt 0,5-7,5 Gew.-% erreicht wird, weiter bevorzugt 0,75-4 Gew.-%, besonders bevorzugt 1-3,5 Gew.-%, bezogen auf die Gesamtmasse des beschichteten Komplexes. Diese Mengen sind für die Beschichtung optimal.

Geeignete Hydroxypropylstärken für die Verwendung in dem erfindungsgemäßen Verfahren sind die vorstehend im Zusammenhang mit einer Zusammensetzung beschriebenen Stärken.

Um das Coating der Cyclodextrin-Komplexe zu begünstigen kann der pH-Wert entsprechend angepasst werden.

In bevorzugten Ausführungsformen der Erfindung werden der Suspension beschichteter (und ggf. homogenisierter) Cyclodextrin-Komplexe weiterhin ein oder mehrere Trägerstoffe bzw. Lyoprotektoren wie Saccharide zugegeben, insbesondere in Form von Maltodextrin. Diese erleichtern die Trocknung der beschichteten Cyclodextrin-Komplexe und verbessern ihre Stabilität. Saccharide können aus der Gruppe ausgewählt werden, die Maltodextrin, Glucose, Saccharose, Fructose, Maltose, Lactose, Galactose, Trehalose, Mannose und Kombinationen davon umfasst. Bevorzugt ist der Einsatz von Stärkehydrolysaten mit einem Dextroseäquivalent (DE) von 3-50, besonders bevorzugt mit einem DE von 5-30, am besten von 10-20. Der Gewichtsanteil der Trägerstoffe bzw. Lyoprotektoren, insbesondere der Saccharide kann, bezogen auf die Gesamtmasse des beschichteten Komplexes, zusätzlich insgesamt 5-50 Gew.-% betragen, bevorzugt 10-40 Gew.-%, bevorzugt 12,5-35% besonders bevorzugt 15-30Gew.-%.

In Schritt (c) wird die in Schritt (b) erhaltene Suspension schließlich getrocknet, beispielsweise durch Sprühtrocknen, Gefriertrocknung, Vakuumbandtrocknung oder Sprühgranulierung.

Abhängig von einer gewünschten Verwendung der beschichteten Cyclodextrin-Komplexe können sie zu einer Zusammensetzung für die orale Verabreichung weiterverarbeitet werden. Hierzu kann die Zusammensetzung beispielsweise als Tablette, Kapsel, Kau-, Schmelz- oder Brausetablette, als Granulat, als Pulver, in Lösung für die orale Einnahme, als Dragee, als Sublingual/Buccaltablette sowie als Schmelzfilm formuliert werden.

Die nachfolgenden Beispiele und die Figuren sollen die Erfindung weiter veranschaulichen. Die Erfindung ist jedoch nicht auf diese konkreten Beispiele beschränkt, sondern sie erstreckt sich auf den gesamten Umfang der Ansprüche.

### Figuren

- **Fig.1**: Reaktionsschema der Derivatisierung von Stärke mit Propylenoxid

### Beispiele

Zur Bestimmung der optimalen Formulierung für Bioverfügbarkeit, Löslichkeit und Dissolution wurden zunächst verschiedene modifizierte Stärken bezüglich der Viskosität charakterisiert, sowie Formulierungen enthaltend die schwerlöslichen Wirkstoffe Curcumin, Quercetin, Boswelliasäuren, Berberin und Silibinin hergestellt. Nach anschließender Charakterisierung wurde die Sättigungslöslichkeit und Freisetzung in verschiedenen Medien ermittelt. Die Konzentrationsangaben in den Beispielen beziehen sich jeweils auf die Gesamtzusammensetzung.

### 1. Charakterisierung der Stärken (Viskosität)

Verschiedene modifizierte Stärken (Acetyl- [E1420], Natriumoctenylsuccinat- [E1450] & Hydroxypropylstärken [E1440] sowie Hydroxypropyldistärkephosphat [E1442]) wurden auf die Viskosität untersucht. Hierzu wurden die getrockneten Stärken auf einem Magnetrührer gelöst (20g Stärke + 180ml dest. Wasser), um eine 10 Gew.-% Lösung herzustellen. Diese Lösung wurde anschließend mittels Brookfield DVPlus Viskosimeter (DVPLLV) bei 25°C vermessen.

### 10 Gew.-% Natriumoctenylsuccinatstärken (E1450, OSA-Stärke):

| **Stärke/kommerzieller Name** | **Kürzel** | **Viskosität bei 25°C [mPas]** | **Spindel** |
|---|---|---|---|
| Hi-Caps 100 (Ingredion) | HC100 | 2-5 | Spindle LV-1 |
| Cleargum CO03 (Roquette) | CO03 | 3-8 | Spindle LV-1 |
| Purity Gum Ultra (Ingredion) | PGU | 50-75 | Spindle LV-1 |
| N-Creamer 2230 (Ingredion) | NC2230 | 8.000 - 15.000 | Spindle LV-3 |

### 10 Gew.-% Hydroxypropylstärken (E1440, HP-Stärke):

| **Stärke/kommerzieller Name** | **Kürzel** | **Viskosität bei 25°C [mPas]** | **Spindel** |
|---|---|---|---|
| Lycoat RS 780 (Roquette) | RS780 | 8-13 | Spindle LV-1 |
| Lycoat RS 720 (Roquette) | RS720 | 25-50 | Spindle LV-1 |
| HPS-T301 (WOTAlchem) | T301 | 600-1.800 | Spindle LV-2 |
| FloTexa-TS-57900 (Angel Starch) | FTTS | 2.200-5.400 | Spindle LV-3 |

### 10 Gew.-% Hydroxypropyldistärkephosphat (E1442 HDP-Stärke):

| **Stärke/kommerzieller Name** | **Kürzel** | **Viskosität bei 25°C [mPas]** | **Spindel** |
|---|---|---|---|
| Purity 87 (Ingredion) | P87 | 400-1300 | Spindle LV-2 |
| Prejel VA 70 (Avebe) | PVA70 | 6.500-12.000 | Spindle LV-3 |

### 10 Gew.-% Acetylstärken (E1420, AS-Stärke):

| **Stärke/kommerzieller Name** | **Kürzel** | **Viskosität bei 25°C [mPas]** | **Spindel** |
|---|---|---|---|
| FROSIN TM-MS-CWS (Angel Starch) | FTM | 40-160 | Spindle LV-1 |
| FROSIN-PS (Angel Starch) | FPS | 1.800-5.000 | Spindle LV-3 |

### 2. Wirkstoffformulierungen mit Curcumin

**Materialien:** Curcumin (98%, Thermo Fisher Scientific), alpha-Cyclodextrin (CAVAMAX W6, Wacker Chemie), beta-Cyclodextrin (CAVAMAX W7, Wacker Chemie), gamma-Cyclodextrin (CAVAMAX W8, Wacker Chemie), Phosphatidylcholin (96%, LIPOID H100, Lipoid), Maltodextrin (GLUCIDEX DE 19, Roquette), NaOH (Qualität entsprechend Ph.Eur., generisch), HCl (rauchend 32%, generisch), modifizierte Stärken (siehe 1. Charakterisierung der Stärken)

### 2.1 Einfluss der modifizierten Stärke

Es wurden zur Abschätzung des optimalen Stärketyps pulverförmige Curcuminkomplexe mit folgender finaler Komposition hergestellt:

| **Bestandteil** | **Gewichtsanteil [Gew.-%]** |
|---|---|
| Curcumin | 10 |
| Gamma-Cyclodextrin | 70 |
| Phosphatidylcholin | 2,5 |
| Modifizierte Stärke | 2,5 |
| Maltodextrin | 15 |

Hierzu wurde zunächst 70 g gamma-Cyclodextrin (CAVAMAX W8) sowie 10g Curcumin in 900 ml destilliertem Wasser mit einem High Shear Homogenisator (IKA Ultra Turrax T 18) bei 5000 rpm dispergiert und anschließend mit NaOH auf einen pH-Wert von 10-11 eingestellt. Zur Komplexbildung wurde die Suspension für weitere 30 min bei 5000 rpm dispergiert und der pH der Suspension anschließend durch Zugabe HCl-Lösung unter leichtem Rühren neutralisiert (finaler pH-Wert 4,5). Hiernach wurde 2,5 g modifizierte Stärke (HC100, CO03, PGU, NC2230, RS780, RS720, T301, FTTS, P87, PVA70, FTM oder FPS) sowie 2,5 g Phosphatidylcholin (Lipoid H100) unter Rühren homogen dispergiert.

Anschließend wurden die Suspensionen für 20 Minuten mit einer Kugelmühle (WAB, DYNO-MILL Research Lab, 65% Füllung mit 0,3-0,4 mm Zirkoniabeads, 6 m/s Tip Speed) in Kreisfahrweise vermahlen und anschließend gewogen. Anschließend wurden 15g Maltodextrin (GLUCIDEX 19) - korrigiert um die Ausbeute (Ausbeute in g/theoretische Ausbeute in g x 15 g) - eingewogen und in der vermahlenen Suspension und Rühren gelöst.

Die Suspensionen wurden zuletzt mit einem Laborsprühtrockner (Büchi B-290, 5ml/min Flow, Inlet Temperatur 130°C, 800l/h Sprühgas, 35 m3/h Aspirator) getrocknet.

Zur Referenz wurde ebenfalls ein nativer Gamma-Cyclodextrin/Curcumin Komplex (Nativ GCD) hergestellt, wobei zunächst nach demselben Verfahren vorgegangen wurde: 70 g gamma-Cyclodextrin (CAVAMAX W8) sowie 10 g Curcumin in 900 ml destilliertem Wasser mit einem High Shear Homogenisator (IKA Ultra Turrax T 18) bei 5000 rpm dispergiert und anschließend mit NaOH auf einen pH-Wert von 10,7 eingestellt. Zur Komplexbildung wurde die Suspension für weitere 30 min bei 5000rpm dispergiert und der pH der Suspension anschließend durch Zugabe HCl-Lösung unter leichtem Rühren neutralisiert (finaler pH-Wert 4,8) und anschließend mit dem Sprühtrockner unter Anwendung derselben Sprühparameter getrocknet.

Die pulverförmigen Komplexe wurden bezüglich Gesamtausbeute, Trocknungsverlust (Halogentrockner), Partikelgröße in Lösung (D50-Wert, Laserbeugung) sowie Curcumingehalt (HPLC) charakterisiert.

Zur Messung der Partikelgröße in Lösung wurde ein Mastersizer 3000 (Malvern Panalytical) mit Hydro MV Nassdispergierungselement genutzt. Hierzu wurden 20mg Probe, genau gewogen, in 120ml Wasser mit einem Tropfen Igepal-Lösung für 10 min mittels leichten Ultraschalls dispergiert, um die Partikel zu vereinzeln und dann vermessen (Auswertung mittels Mie-Theorie).

| Komplex | Stärketyp | Verwendete Stärke | Ausbeute [g] | Trocknungsverlust [%] | Curcumin Gehalt [Gew.-%] | D50-Wert [µm] |
|---|---|---|---|---|---|---|
| CC1A | OSA | HC100 | 58,4 | 4,8 | 10,2 | 2,719 |
| CC1B | OSA | CO03 | 73,7 | 4,5 | 10,0 | 4,721 |
| CC1C | OSA | PGU | 64,6 | 5,3 | 9,7 | 3,219 |
| CC1D | OSA | NC2230 | 34,7 | 6,5 | 10,1 | 12,892 |
| CC2A | HP | RS780 | 70,6 | 5,7 | 10,1 | 1,163 |
| CC2B | HP | RS720 | 69,8 | 5,2 | 10,0 | 1,340 |
| CC2C | HP | T301 | 65,2 | 6,2 | 9,9 | 6,621 |
| CC2D | HP | FTTS | 25,3 | 7,7 | 10,2 | 6,821 |
| CC3A | HDP | P87 | 61,5 | 6,6 | 9,7 | 8,941 |
| CC3B | HDP | PVA70 | 28,5 | 7,8 | 10,0 | 15,997 |
| CC4A | AS | FTM | 55,7 | 7,2 | 10,1 | 7,893 |
| CC4B | AS | FPS | 47,8 | 6,8 | 9,9 | 21,783 |
| Nativ GCD | keine | keine | 58,3 | 6,9 | 12,3 | 11,921 |
| Curcumin | - | - | - | 0,22 | 98,9 | 24,6 |

Zur Messung der Sättigungslöslichkeit wurden Proben enthaltend 25mg Curcumin genau abgewogen und in 100 ml biorelevantem Puffer (FASSIF-V2, Biorelevant: pH 6.5, Pufferkapazität 10 mmol/delta pH/L enthaltend 3mM Natrium-Taurocholat; 0,2 mM Lecithin; 19,12 mM Maleinsäure 34,8 mM; Natriumhydroxid; 68,62 mM Natriumchlorid, entspricht bzgl. Zusammensetzung, pH-Wert, Ionenstärke sowie Pufferkapazität dem physiologischen Darmmedium) dispergiert und anschließend für 2h unter leichtem Schütteln inkubiert. Hiernach wurden die Proben mittels Spritzenfilter filtriert und die Curcuminkonzentration des Filtrats mittels HPLC bestimmt. Die Versuche wurden jeweils in Triplikaten angesetzt, die Durchschnittswerte sind tabellarisch dargestellt. Reines Curcumin wurde als Kontrolle mitgeführt.

| Komplex | Stärketyp | Verwendete Stärke | Volumen FASSIF-V2 [ml] | Sättigungslöslichkeit Curcumin in FASSIF-V2 [µg/ml] | Sättigungslöslichkeit Curcumin in FASSIF-V2 [%] |
|---|---|---|---|---|---|
| CC1A | OSA | HC100 | 100 | 64,2 | 25,68 |
| CC1B | OSA | CO03 | 100 | 58,9 | 23,56 |
| CC1C | OSA | PGU | 100 | 48,9 | 19,56 |
| CC1D | OSA | NC2230 | 100 | 44,6 | 17,84 |
| CC2A | HP | RS780 | 100 | 158,2 | 63,28 |
| CC2B | HP | RS720 | 100 | 152,8 | 61,12 |
| CC2C | HP | T301 | 100 | 83,9 | 33,56 |
| CC2D | HP | FTTS | 100 | 69,3 | 27,72 |
| CC3A | HDP | P87 | 100 | 38,4 | 15,36 |
| CC3B | HDP | PVA70 | 100 | 32,4 | 12,96 |
| CC4A | AS | FTM | 100 | 41,2 | 16,48 |
| CC4B | AS | FPS | 100 | 35,8 | 14,32 |
| Nativ GCD | keine | keine | 100 | 54,6 | 21,84 |
| Curcumin | - | - | 100 | 1,23 | 0,492 |

Anschließend wurde die in-vitro Freisetzung nach Vorgaben des Arzneibuches (Ph. Eur. 11.0 Kapitel 2.9.3. Freisetzung fester Darreichungsformen) untersucht. Hierzu wurde der USP Apparatus II (Paddle/Blattrührer) ausgewählt, die Umdrehungsgeschwindigkeit betrug 75rpm, V=500ml Fassif-V2 Medium, T= 37°C.

Es wurden Proben enthaltend 50mg Curcumin genau eingewogen, entsprechend einer Zielkonzentration von 100µg/ml Curcumin. Nachdem das Medium auf Zieltemperatur gebracht und die Proben in die Gefäße gegeben wurden, erfolgte der Probenzug nach 15, 30, 60 und 120 Minuten, wobei die Proben mittels Spritzenfilter filtriert und die Curcuminoid-Konzentration mittels HPLC bestimmt wurde. Entnommenes Probenvolumen wurde sofort nach Entnahme durch frisches, vortemperiertes FASSIF-V2 Medium ersetzt. Die Versuche wurden jeweils in Triplikaten angesetzt, die Durchschnittswerte der Freisetzung (Q in %) sind tabellarisch dargestellt.

| Komplex | Stärketyp | Verwendete Stärke | Q nach 15min [%] | Q nach 30min [%] | Q nach 60min [%] | Q nach 120min [%] |
|---|---|---|---|---|---|---|
| CC1A | OSA | HC100 | 40,2 | 34,46 | 27,56 | 20,85 |
| CC1B | OSA | CO03 | 31,74 | 33,17 | 29,90 | 24,57 |
| CC1C | OSA | PGU | 35,6 | 37,5 | 25,6 | 18,4 |
| CC1D | OSA | NC2230 | 22,3 | 25,6 | 28,4 | 20,8 |
| CC2A | HP | RS780 | 72,6 | 77,4 | 75,9 | 65,1 |
| CC2B | HP | RS720 | 69,2 | 74,5 | 68,7 | 59,7 |
| CC2C | HP | T301 | 50,6 | 61,2 | 45,1 | 36,8 |
| CC2D | HP | FTTS | 32,1 | 36,7 | 28,9 | 25,6 |
| CC3A | HDP | P87 | 16,7 | 21,3 | 18,5 | 13,8 |
| CC3B | HDP | PVA70 | 9,6 | 15,6 | 16,9 | 10,7 |
| CC4A | AS | FTM | 26,8 | 32,4 | 23,5 | 18,3 |
| CC4B | AS | FPS | 18,7 | 22,3 | 16,7 | 11,2 |
| Nativ GCD | keine | keine | 39,4 | 41,5 | 32,6 | 18,4 |
| Curcumin | - | - | 0,11 | 0,16 | 0,25 | 0,46 |

Lediglich Hydroxypropylstärke und insbesondere niedrigviskose Hydroxypropylstärke in Kombination mit Phosphatidylcholin erzielt eine deutliche Steigerung der Sättigungslöslichkeit sowie der Freisetzung im Vergleich zu einem nativem gamma-Cyclodextrin/Curcumin Komplex.

### 2.2 Einfluss des Cyclodextrin-Typs

Um den Einfluss des Cyclodextrintyps zu untersuchen, wurden Komplexe entsprechend dem Vorgehen aus 2.1. mit der besten modifizierten Stärke (Lycoat RS780) hergestellt, welche jedoch statt gamma-Cyclodextrin alpha- bzw. beta-Cyclodextrin enthielten.

### Komposition mit alpha-Cyclodextrin (CC5A):

| **Bestandteil** | **Gewichtsanteil [Gew.-%]** |
|---|---|
| Curcumin | 10 |
| Alpha-Cyclodextrin | 70 |
| Phosphatidylcholin | 2,5 |
| Modifizierte Stärke (Lycoat RS780) | 2,5 |
| Maltodextrin | 15 |

### Komposition mit beta-Cyclodextrin (CC6A):

| **Bestandteil** | **Gewichtsanteil [Gew.-%]** |
|---|---|
| Curcumin | 10 |
| Beta-Cyclodextrin | 70 |
| Phosphatidylcholin | 2,5 |
| Modifizierte Stärke (Lycoat RS780) | 2,5 |
| Maltodextrin | 15 |

Zur Referenz wurden ebenfalls native alpha/beta-Cyclodextrin:Curcumin Komplex (Nativ ACD und Nativ BCD) hergestellt, wobei zunächst nach demselben Verfahren vorgegangen wurde:
70g alpha-Cyclodextrin (CAVAMAX W6) bzw. 70g beta-Cyclodextrin (CAVAMAX W7) sowie 10g Curcumin in 900ml destilliertem Wasser mit einem High Shear Homogenisator (IKA Ultra Turrax T 18) bei 5000 rpm dispergiert und anschließend mit NaOH auf einen pH Wert von 10-11 eingestellt. Zur Komplexbildung wurde die Suspension für weitere 30min bei 5000rpm dispergiert und der pH der Suspension anschließend durch Zugabe HCl-Lösung unter leichtem Rühren neutralisiert (finaler pH-Wert 4-5) und anschließend mit dem Sprühtrockner unter Anwendung derselben Sprühparameter wie in 3.2.1. beschrieben getrocknet.

Diese Komplexe wurden daraufhin auf Sättigungslöslichkeit und in-vitro Freisetzung entsprechend dem bereits beschriebenen Vorgehen getestet.

Zur Messung der Sättigungslöslichkeit wurden Proben enthaltend 25mg Curcumin genau abgewogen und in 100ml biorelevantem Puffer (FASSIF-V2, Biorelevant: pH 6.5, Pufferkapazität 10mmol/delta pH/L enthaltend 3mM Natrium-Taurocholat; 0,2mM Lecithin; 19,12mM Maleinsäure 34,8mM; Natriumhydroxid; 68,62mM Natriumchlorid, entspricht bzgl. Zusammensetzung, pH-Wert, Ionenstärke sowie Pufferkapazität dem physiologischen Darmmedium) dispergiert und anschließend für 2h unter leichtem Schütteln inkubiert. Hiernach wurden die Proben mittels Spritzenfilter filtriert und die Curcuminkonzentration des Filtrats mittels HPLC bestimmt. Die Versuche wurden jeweils in Triplikaten angesetzt, die Durchschnittswerte sind tabellarisch dargestellt.

Zur besseren Übersicht wurden die Werte für den nativen Gamma-Cyclodextrin/Curcumin Komplex (nativ GCD), reinem Curcumin, sowie dem besten Komplex aus 3.2.1. mit LycoatRS780 (CC2A) mit in der Tabelle aufgeführt:

| Komplex | CD | Verwendete Stärke | Volumen FASSIF-V2 [ml] | Sättigungslöslichkeit Curcumin in FASSIF-V2 [µg/ml] | Sättigungslöslichkeit Curcumin in FASSIF-V2 [%] |
|---|---|---|---|---|---|
| CC5A | alpha | RS780 | 100 | 18,5 | 7,4 |
| Nativ ACD | alpha | keine | 100 | 22,3 | 8,9 |
| CC6A | beta | RS780 | 100 | 38,2 | 15,3 |
| Nativ BCD | beta | keine | 100 | 35,5 | 14,2 |
| CC2A | gamma | RS780 | 100 | 158,2 | 63,28 |
| Nativ GCD | gamma | keine | 100 | 54,6 | 21,84 |
| Curcumin | - | - | 100 | 1,23 | 0,492 |

Anschließend wurde entsprechend dem Vorgehen in 3.2.1. die in-vitro Freisetzung nach Vorgaben des Arzneibuches (Ph. Eur. 11.0 Kapitel 2.9.3. Freisetzung fester Darreichungsformen) untersucht. Hierzu wurde der USP Apparatus II (Paddle/Blattrührer) ausgewählt, die Umdrehungsgeschwindigkeit betrug 75rpm, V=500ml Fassif-V2 Medium, T= 37°C.

Es wurden Proben enthaltend 50mg Curcumin genau eingewogen, entsprechend einer Zielkonzentration von 100µg/ml Curcumin. Nachdem das Medium auf Zieltemperatur gebracht und die Proben in die Gefäße gegeben wurden, erfolgte der Probenzug nach 15, 30, 60 und 120 Minuten, wobei die Proben mittels Spritzenfilter filtriert und die Curcuminoidkonzentration mittels HPLC bestimmt wurde. Entnommenes Probenvolumen wurde sofort nach Entnahme durch frisches, vortemperiertes FASSIF-V2 Medium ersetzt. Die Versuche wurden jeweils in Triplikaten angesetzt, die Durchschnittswerte der Freisetzung (Q in %) sind tabellarisch dargestellt.

| Komplex | CD | Verwendete Stärke | Q nach 15min [%] | Q nach 30min [%] | Q nach 60min [%] | Q nach 120min [%] |
|---|---|---|---|---|---|---|
| CC5A | alpha | RS780 | 5,8 | 6,3 | 7,8 | 7,4 |
| Nativ ACD | alpha | keine | 4,3 | 5,1 | 8,9 | 10,2 |
| CC6A | beta | RS780 | 14,3 | 15,6 | 16,2 | 16,1 |
| Nativ BCD | beta | keine | 15,9 | 16,2 | 16,3 | 16,7 |
| CC2A | gamma | RS780 | 72,6 | 77,4 | 75,9 | 65,1 |
| Nativ GCD | gamma | keine | 39,4 | 41,5 | 32,6 | 18,4 |
| Curcumin | - | - | 0,11 | 0,16 | 0,25 | 0,46 |

Es konnte nur mit gamma-Cyclodextrin eine signifikante Steigerung der Sättigungslöslichkeit sowie des Dissolutionsverhaltens - sowohl relativ im Vergleich zu dem jeweiligen nativen Komplex als auch absolut - erzielt werden.

### 2.3 Einfluss des Anteils modifizierter Stärke sowie Phosphatidylcholin

Um den Einfluss des Gewichtsanteils der modifizierten Stärke sowie des Phosphatidylcholins zu ermitteln, wurden Komplexe entsprechend dem Vorgehen aus 2.1. mit der besten modifizierten Stärke (Lycoat RS780), Phosphatidylcholin (Lipoid H100) und gamma-Cyclodextrin hergestellt, welche jedoch unterschiedliche Gewichtsanteile der beiden Additive enthielten.

Dabei wurde entweder 2,5% Lycoat RS780 beibehalten und der Anteil Phosphatidylcholin geändert (0,1%, 0,5%, 1%, 4%, 5% 7,5%) oder der Anteil an 2,5% Phosphatidylcholin beibehalten und der Anteil Lycoat RS780 angepasst (0,1%, 0,5%, 1%, 4%, 5% 7,5%) - die Differenzmenge wurde hierbei vom Maltodextrin abgezogen. Darüber hinaus wurden Komplexe mit jeweils 1% RS780 + 1% Phosphatidylcholin sowie 0,5% RS780 + 0,5% Phosphatidlycholin hergestellt. Als abschließende Kontrollen dienten Komplexe mit jeweils nur 2,5% von einem Additiv (RS780 oder Phosphatidylcholin).

### Komplexe enthaltend 2,5% Lycoat RS780 + wechselnde Menge an Phosphatidylcholin:

### Komposition mit 2,5% Lycoat und 0,1% Phosphatidylcholin (CC7A):

| **Bestandteil** | **Gewichtsanteil Gew.-%** |
|---|---|
| Curcumin | 10 |
| Gamma-Cyclodextrin | 70 |
| Phosphatidylcholin | 0,1 |
| Modifizierte Stärke (Lycoat RS780) | 2,5 |
| Maltodextrin | 17,4 |

### Komposition mit 2,5% Lycoat und 0,5% Phosphatidylcholin (CC7B):

| **Bestandteil** | **Gewichtsanteil Gew.-%** |
|---|---|
| Curcumin | 10 |
| Gamma-Cyclodextrin | 70 |
| Phosphatidylcholin | 0,5 |
| Modifizierte Stärke (Lycoat RS780) | 2,5 |
| Maltodextrin | 17 |

### Komposition mit 2,5% Lycoat und 1% Phosphatidylcholin (CC7C):

| **Bestandteil** | **Gewichtsanteil Gew.-%** |
|---|---|
| Curcumin | 10 |
| Gamma-Cyclodextrin | 70 |
| Phosphatidylcholin | 1 |
| Modifizierte Stärke (Lycoat RS780) | 2,5 |
| Maltodextrin | 16,5 |

### Komposition mit 2,5% Lycoat und 4% Phosphatidylcholin (CC7D):

| **Bestandteil** | **Gewichtsanteil Gew.-%** |
|---|---|
| Curcumin | 10 |
| Gamma-Cyclodextrin | 70 |
| Phosphatidylcholin | 4 |
| Modifizierte Stärke (Lycoat RS780) | 2,5 |
| Maltodextrin | 13,5 |

### Komposition mit 2,5% Lycoat und 5% Phosphatidylcholin (CC7E):

| **Bestandteil** | **Gewichtsanteil Gew.-%** |
|---|---|
| Curcumin | 10 |
| Gamma-Cyclodextrin | 70 |
| Phosphatidylcholin | 5 |
| Modifizierte Stärke (Lycoat RS780) | 2,5 |
| Maltodextrin | 12,5 |

### Komposition mit 2,5% Lycoat und 7,5% Phosphatidylcholin (CC7F):

| **Bestandteil** | **Gewichtsanteil Gew.-%** |
|---|---|
| Curcumin | 10 |
| Gamma-Cyclodextrin | 70 |
| Phosphatidylcholin | 7,5 |
| Modifizierte Stärke (Lycoat RS780) | 2,5 |
| Maltodextrin | 10 |

### Komplexe enthaltend 2,5% Phosphatidylcholin + wechselnde Menge an Lycoat RS780:

### Komposition mit 2,5% Phosphatidylcholin und 0,1% Lycoat RS780 (CC8A):

| **Bestandteil** | **Gewichtsanteil Gew.-%** |
|---|---|
| Curcumin | 10 |
| Gamma-Cyclodextrin | 70 |
| Phosphatidylcholin | 2,5 |
| Modifizierte Stärke (Lycoat RS780) | 0,1 |
| Maltodextrin | 17,4 |

### Komposition mit 2,5% Phosphatidylcholin und 0,5% Lycoat RS780 (CC8B):

| **Bestandteil** | **Gewichtsanteil Gew.-%** |
|---|---|
| Curcumin | 10 |
| Gamma-Cyclodextrin | 70 |
| Phosphatidylcholin | 2,5 |
| Modifizierte Stärke (Lycoat RS780) | 0,5 |
| Maltodextrin | 17 |

### Komposition mit 2,5% Phosphatidylcholin und 1% Lycoat RS780 (CC8C):

| **Bestandteil** | **Gewichtsanteil Gew.-%** |
|---|---|
| Curcumin | 10 |
| Gamma-Cyclodextrin | 70 |
| Phosphatidylcholin | 2,5 |
| Modifizierte Stärke (Lycoat RS780) | 1 |
| Maltodextrin | 16,5 |

### Komposition mit 2,5% Phosphatidylcholin und 4% Lycoat RS780 (CC8D):

| **Bestandteil** | **Gewichtsanteil Gew.-%** |
|---|---|
| Curcumin | 10 |
| Gamma-Cyclodextrin | 70 |
| Phosphatidylcholin | 2,5 |
| Modifizierte Stärke (Lycoat RS780) | 4 |
| Maltodextrin | 13,5 |

### Komposition mit 2,5% Phosphatidylcholin und 5% Lycoat RS780 (CC8E):

| **Bestandteil** | **Gewichtsanteil Gew.-%** |
|---|---|
| Curcumin | 10 |
| Gamma-Cyclodextrin | 70 |
| Phosphatidylcholin | 2,5 |
| Modifizierte Stärke (Lycoat RS780) | 5 |
| Maltodextrin | 12,5 |

### Komposition mit 2,5% Phosphatidylcholin und 7,5% Lycoat RS780 (CC8F):

| **Bestandteil** | **Gewichtsanteil Gew.-%** |
|---|---|
| Curcumin | 10 |
| Gamma-Cyclodextrin | 70 |
| Phosphatidylcholin | 2,5 |
| Modifizierte Stärke (Lycoat RS780) | 7,5 |
| Maltodextrin | 10 |

### Komplexe enthaltend identische Anteile Phosphatidylcholin + Lycoat RS780 (je 0,5% bzw 1%):

### Komposition mit 0,5% Phosphatidylcholin und 0,5% Lycoat RS780 (CC9A):

| **Bestandteil** | **Gewichtsanteil Gew.-%** |
|---|---|
| Curcumin | 10 |
| Gamma-Cyclodextrin | 70 |
| Phosphatidylcholin | 0,5 |
| Modifizierte Stärke (Lycoat RS780) | 0,5 |
| Maltodextrin | 19 |

### Komposition mit 1% Phosphatidylcholin und 1% Lycoat RS780 (CC9B):

| **Bestandteil** | **Gewichtsanteil Gew.-%** |
|---|---|
| Curcumin | 10 |
| Gamma-Cyclodextrin | 70 |
| Phosphatidylcholin | 1 |
| Modifizierte Stärke (Lycoat RS780) | 1 |
| Maltodextrin | 18 |

### Kontroll Komplexe enthaltend nur ein Additiv zu 2,5% (Phosphatidylcholin oder Lycoat RS780):

### Komposition mit 2,5% Phosphatidylcholin und 0% Lycoat RS780 (CC10A):

| **Bestandteil** | **Gewichtsanteil Gew.-%** |
|---|---|
| Curcumin | 10 |
| Gamma-Cyclodextrin | 70 |
| Phosphatidylcholin | 2,5 |
| Modifizierte Stärke (Lycoat RS780) | 0 |
| Maltodextrin | 17,5 |

### Komposition mit 0% Phosphatidylcholin und 2,5% Lycoat RS780 (CC10B):

| **Bestandteil** | **Gewichtsanteil Gew.-%** |
|---|---|
| Curcumin | 10 |
| Gamma-Cyclodextrin | 70 |
| Phosphatidylcholin | 0 |
| Modifizierte Stärke (Lycoat RS780) | 2,5 |
| Maltodextrin | 17,5 |

Zur Messung der Sättigungslöslichkeit wurden entsprechend dem Vorgehen beschrieben in 2.1. Proben enthaltend 25mg Curcumin genau abgewogen und in 100ml biorelevantem Puffer (FASSIF-V2, Biorelevant: pH 6.5, Pufferkapazität 10mmol/delta pH/L enthaltend 3mM Natrium-Taurocholat; 0,2mM Lecithin; 19,12mM Maleinsäure 34,8mM; Natriumhydroxid; 68,62mM Natriumchlorid, entspricht bzgl. Zusammensetzung, pH-Wert, Ionenstärke sowie Pufferkapazität dem physiologischen Darmmedium) dispergiert und anschließend für 2h unter leichtem Schütteln inkubiert. Hiernach wurden die Proben mittels Spritzenfilter filtriert und die Curcuminkonzentration des Filtrats mittels HPLC bestimmt. Die Versuche wurden jeweils in Triplikaten angesetzt, die Durchschnittswerte sind tabellarisch dargestellt.

Zur besseren Übersicht wurden die Werte für Komplex mit gamma-Cyclodextrin enthaltend jeweils 2,5% von Lycoat RS780 und Phosphatidylcholin (CC2A) mit in der Tabelle aufgeführt:

| Komplex | Anteil RS780 [%] | Anteil PC [%] | Volumen FASSIF-V2 [ml] | Sättigungslöslichkeit Curcumin in FASSIF-V2 [µg/ml] | Sättigungslöslichkeit Curcumin in FASSIF-V2 [%] |
|---|---|---|---|---|---|
| CC7A | 2,5 | 0,1 | 100 | 78,6 | 31,44 |
| CC7B | 2,5 | 0,5 | 100 | 137,4 | 54,96 |
| CC7C | 2,5 | 1 | 100 | 142,6 | 57,04 |
| CC7D | 2,5 | 4 | 100 | 118,7 | 47,48 |
| CC7E | 2,5 | 5 | 100 | 104,8 | 41,92 |
| CC7F | 2,5 | 7,5 | 100 | 81,2 | 32,48 |
| CC8A | 0,1 | 2,5 | 100 | 130,6 | 52,24 |
| CC8B | 0,5 | 2,5 | 100 | 160,4 | 64,16 |
| CC8C | 1 | 2,5 | 100 | 183,7 | 73,48 |
| CC8D | 4 | 2,5 | 100 | 109,2 | 43,68 |
| CC8E | 5 | 2,5 | 100 | 89,4 | 35,76 |
| CC8F | 7,5 | 2,5 | 100 | 72,2 | 28,88 |
| CC9A | 0,5 | 0,5 | 100 | 120,3 | 48,12 |
| CC9B | 1 | 1 | 100 | 136,7 | 54,68 |
| CC2A | 2,5 | 2,5 | 100 | 158,2 | 63,28 |
| CC10A | 0 | 2,5 | 100 | 65,2 | 26,08 |
| CC10B | 2,5 | 0 | 100 | 52,3 | 20,92 |
| Nativ GCD | gamma | keine | 100 | 54,6 | 21,84 |
| Curcumin | - | - | 100 | 1,23 | 0,492 |

### 3. Wirkstoffformulierungen mit Silybin

**Material:** Silybin (98%, Fengrui Biotechnology Co., Ltd.), alpha-Cyclodextrin (CAVAMAX W6, Wacker Chemie), beta-Cyclodextrin (CAVAMAX W7, Wacker Chemie), gamma-Cyclodextrin (CAVAMAX W8, Wacker Chemie), Phosphatidylcholin (96%, LIPOID H100, Lipoid), Maltodextrin (GLUCIDEX DE 19, Roquette), modifizierte Stärken (siehe 1. Charakterisierung der Stärken).

### 3.1 Einfluss der modifizierten Stärke

Es wurden zur Abschätzung des optimalen Stärketyps pulverförmige Silybinkomplexe mit folgender finaler Komposition hergestellt:

| **Bestandteil** | **Gewichtsanteil Gew.-%** |
|---|---|
| Silybin | 32 |
| Gamma-Cyclodextrin | 50 |
| Phosphatidylcholin | 2 |
| Modifizierte Stärke | 1 |
| Maltodextrin | 15 |

Hierzu wurden zunächst 75g gamma-Cyclodextrin (CAVAMAX W8) sowie 48g Silybin in 850ml destilliertem Wasser mit einem High Shear Homogenisator (IKA Ultra Turrax T 18) bei 7000 rpm für 1-2 Minuten eingerührt und anschließend zur Komplexbildung für weitere 30min bei 5000rpm dispergiert. Hiernach wurde 1,5g modifizierte Stärke (HC100, NC2230, RS780, T301, FTTS, P87, PVA70, FTM) sowie 3g Phosphatidylcholin (Lipoid H100) unter Rühren homogen dispergiert.

Anschließend wurde die Lösung mittels Hochdruckhomogenisator (GEA, PandaPlus 1000) bei 300 bar in 2 Zyklen homogenisiert und anschließend gewogen.

Anschließend wurden 22,5g Maltodextrin (GLUCIDEX 19) - korrigiert um die Ausbeute (Ausbeute in g/theoretische Ausbeute in g x 22,5g) - eingewogen und in der vermahlenen Suspension und Rühren gelöst.

Die Suspensionen wurden zuletzt mit einem Laborsprühtrockner (Büchi B-290, 5ml/min Flow, Inlet Temperatur 140°C, 600l/h Sprühgas, 35 m3/h Aspirator) getrocknet.

Zur Referenz wurde ebenfalls ein nativer Gamma-Cyclodextrin/Silybinn Komplex (Nativ GCD) hergestellt, wobei zunächst nach demselben Verfahren vorgegangen wurde: 75g gamma-Cyclodextrin (CAVAMAX W8) sowie 48g Silybin wurden in 850ml destilliertem Wasser mit einem High Shear Homogenisator (IKA Ultra Turrax T 18) bei 7000 rpm für 1-2 Minuten eingerührt und anschließend zur Komplexbildung für weitere 30min bei 5000rpm dispergiert. Anschließend erfolgte die Trocknung mittels Sprühtrockner unter Anwendung der oben genannten Sprühparameter.

Die pulverförmigen Komplexe wurden bezüglich Trocknungsverlust (Halogentrockner), Partikelgröße in Lösung (D50-Wert, Laserbeugung) sowie Silybingehalt (HPLC) charakterisiert.

Zur Messung der Partikelgröße in Lösung wurde ein Mastersizer 3000 (Malvern Panalytical) mit Hydro MV Nassdispergierungselement genutzt. Hierzu wurden 25mg Probe, genau gewogen, in 120ml Wasser mit einem Tropfen Igepal-Lösung für 10min mittels leichten Ultraschalls dispergiert, um die Partikel zu vereinzeln und dann vermessen (Auswertung mittels Mie-Theorie).

| Komplex | Stärketyp | Verwendete Stärke | Trocknungsverlust [%] | Silybin Gehalt [Gew.-%] | D50-Wert [µm] |
|---|---|---|---|---|---|
| SB1A | OSA | HC100 | 3,3 | 31,7 | 4,821 |
| SB1B | OSA | NC2230 | 6,2 | 32,1 | 16,733 |
| SB2A | HP | RS780 | 4,1 | 31,3 | 3,216 |
| SB2B | HP | T301 | 6,8 | 32,6 | 12,429 |
| SB2C | HP | FTTS | 6,2 | 31,8 | 23,961 |
| SB3A | HDP | P87 | 6,7 | 32,2 | 25,596 |
| SB3B | HDP | PVA70 | 7,8 | 31,2 | 34,821 |
| SB4A | AS | FTM | 5,9 | 31,8 | 18,946 |
| Nativ GCD | keine | keine | 5,6 | 38,7 | 28,393 |
| Silybin | - | - | 0,92 | 98,7 | 18,767 |

Anschließend wurde die in-vitro Freisetzung nach Vorgaben des Arzneibuches (Ph. Eur. 11.0 Kapitel 2.9.3. Freisetzung fester Darreichungsformen) untersucht. Hierzu wurde der USP Apparatus II (Paddle/Blattrührer) ausgewählt, die Umdrehungsgeschwindigkeit betrug 50rpm, V=500ml, T= 37°C. Als Medium wurde ein 0,05M Phosphatpuffer (pH 6.8) + 0,1% SDS gewählt.

Es wurden Proben enthaltend 100mg Silybin genau eingewogen, entsprechend einer Zielkonzentration von 200µg/ml Silybin. Nachdem das Medium auf Zieltemperatur gebracht und die Proben in die Gefäße gegeben wurden, erfolgte der Probenzug nach 15, 30, 60 und 120 Minuten, wobei die Proben mittels Spritzenfilter filtriert und die Silybin-Konzentration mittels HPLC bestimmt wurde. Entnommenes Probenvolumen wurde sofort nach Entnahme durch frisches, vortemperiertes Medium ersetzt. Die Versuche wurden jeweils in Triplikaten angesetzt, die Durchschnittswerte der Freisetzung (Q in %) sind tabellarisch dargestellt.

| Komplex | Stärketyp | Verwendete Stärke | Q nach 15min [%] | Q nach 30min [%] | Q nach 60min [%] | Q nach 120min [%] |
|---|---|---|---|---|---|---|
| SB1A | OSA | HC100 | 11,2 | 13,91 | 15,63 | 16,12 |
| SB1B | OSA | NC2230 | 3,21 | 4,56 | 6,72 | 8,23 |
| SB2A | HP | RS780 | 21,53 | 26,78 | 41,29 | 43,64 |
| SB2B | HP | T301 | 9,89 | 13,92 | 16,93 | 18,93 |
| SB2C | HP | FTTS | 3,91 | 5,82 | 7,92 | 9,48 |
| SB3A | HDP | P87 | 5,62 | 6,82 | 8.92 | 10,32 |
| SB3B | HDP | PVA70 | 2,23 | 3,28 | 4,89 | 5,31 |
| SB4A | AS | FTM | 4,29 | 5,21 | 7,83 | 8,79 |
| Nativ GCD | keine | keine | 8,34 | 12,89 | 13,44 | 13,89 |
| Silybin | - | - | 1,53 | 1,89 | 2,15 | 2,33 |

Lediglich niedrigviskose Hydroxypropylstärke in Kombination mit Phosphatidylcholin erzielt eine deutliche Steigerung der Sättigungslöslichkeit sowie der Freisetzung im Vergleich zu einem nativem gamma Cyclodextrin/Silybin Komplex.

### 3.2 Einfluss des Cyclodextrin-Typs

Um den Einfluss des Cyclodextrintyps zu untersuchen, wurden Komplexe entsprechend dem Vorgehen aus 3.1. mit der besten modifizierten Stärke (Lycoat RS780) hergestellt, welche jedoch statt gamma-Cyclodextrin alpha- bzw. beta-Cyclodextrin enthielten.

### Komposition mit alpha-Cyclodextrin (SB5A):

| **Bestandteil** | **Gewichtsanteil Gew.-%** |
|---|---|
| Silybin | 32 |
| Alpha-Cyclodextrin | 50 |
| Phosphatidylcholin | 2 |
| Modifizierte Stärke (Lycoat RS780) | 1 |
| Maltodextrin | 15 |

### Komposition mit beta-Cyclodextrin (SB6A):

| **Bestandteil** | **Gewichtsanteil Gew.-%** |
|---|---|
| Silybin | 32 |
| Beta-Cyclodextrin | 50 |
| Phosphatidylcholin | 2 |
| Modifizierte Stärke (Lycoat RS780) | 1 |
| Maltodextrin | 15 |

Zur Referenz wurden ebenfalls native alpha/beta-Cyclodextrin:Silybin Komplex (Nativ ACD und Nativ BCD) hergestellt, wobei zunächst nach demselben Verfahren vorgegangen wurde:
75g alpha-Cyclodextrin (CAVAMAX W6) bzw. 75g beta-Cyclodextrin (CAVAMAX W7) sowie 48g Silybin in 850ml destilliertem Wasser mit einem High Shear Homogenisator (IKA Ultra Turrax T 18) bei 7000 rpm für 1-2 Minuten eingerührt und anschließend zur Komplexbildung für weitere 30min bei 5000rpm dispergiert. Anschließend erfolgte die Trocknung mittels Sprühtrockner unter Anwendung der oben genannten Sprühparameter.

Zur Messung der Sättigungslöslichkeit wurden Proben enthaltend 50mg Silybin genau abgewogen und in 100ml 0,05M Phosphatpuffer pH 6.8 + 0,1% SDS dispergiert und anschließend für 2h unter leichtem Schütteln inkubiert. Hiernach wurden die Proben mittels Spritzenfilter filtriert und die Silybinkonzentration des Filtrats mittels HPLC bestimmt. Die Versuche wurden jeweils in Triplikaten angesetzt, die Durchschnittswerte sind tabellarisch dargestellt.

Die Versuche wurden ebenfalls mit dem besten Komplex aus 3.3.1 (SB2A mit RS780) sowie nativem Silybin/gamma Cyclodextrin Komplex (Nativ GCD) durchgeführt:

| Komplex | CD | Verwendete Stärke | Volumen FASSIF-V2 [ml] | Sättigungslöslichkeit Silybin in PBS pH 6.8 +0,1% SDS [µg/ml] | Sättigungslöslichkeit Silybin in PBS pH 6.8 +0,1% SDS [%] |
|---|---|---|---|---|---|
| SB5A | alpha | RS780 | 100 | 22,55 | 4,51 |
| Nativ ACD | alpha | keine | 100 | 19,45 | 3,89 |
| SB6A | beta | RS780 | 100 | 33,9 | 6,78 |
| Nativ BCD | beta | keine | 100 | 29,1 | 5,82 |
| SB2A | gamma | RS780 | 100 | 189,55 | 37,91 |
| Nativ GCD | gamma | keine | 100 | 44,55 | 8,91 |
| Silybin | - | - | 100 | 7,7 | 1,54 |

Es konnte nur mit gamma-Cyclodextrin eine signifikante Steigerung der Sättigungslöslichkeitsowohl relativ im Vergleich zu dem jeweiligen nativen Komplex als auch absolut - erzielt werden.

### 4. Wirkstoffformulierung mit Quercetin

**Material:** Quercetin Dihydrat (95%, Shaanxi Jia He Phytochem Co.,Ltd.), alpha-Cyclodextrin (CAVAMAX W6, Wacker Chemie), beta-Cyclodextrin (CAVAMAX W7, Wacker Chemie), gamma-Cyclodextrin (CAVAMAX W8, Wacker Chemie), Phosphatidylcholin (96%, LIPOID H100, Lipoid), Maltodextrin (GLUCIDEX DE 19, Roquette), Ammoniaklösung (9% Ammoniak, wässrig., generisch), HCl (rauchend 32%, generisch), Lycoat RS780 (Roquette)

### 4.1 Komplexherstellung

Es wurden zur Abschätzung des optimalen Cyclodextrintyps pulverförmige Quercetinkomplexe mit folgender finaler Komposition hergestellt:

| **Bestandteil** | **Gewichtsanteil [Gew.-%]** |
|---|---|
| Quercetin Dihydrat | 16 |
| Cyclodextrin (alpha, beta oder gamma) | 60 |
| Phosphatidylcholin /Lipoid H100 | 3 |
| Lycoat RS780 | 3 |
| Maltodextrin/Glucidex DE 19 | 18 |

Hierzu wurde zunächst 75g Cyclodextrin (CAVAMAX W6 bzw. W7 bzw. W8) sowie 20g Quercetin Dihydrat in 875ml destilliertem Wasser mit einem High Shear Homogenisator (IKA Ultra Turrax T 18) bei 5000 rpm dispergiert und anschließend mit wässriger Ammoniaklösung auf einen pH-Wert von 9 eingestellt. Zur Komplexbildung wurde die Suspension für weitere 30min bei 5000rpm dispergiert und der pH der Suspension anschließend durch Zugabe HCl-Lösung unter leichtem Rühren neutralisiert (finaler pH-Wert 2,5-3,5). Hiernach wurde 3,75g modifizierte Stärke (RS780) sowie 3,75g Phosphatidylcholin (Lipoid H100) unter Rühren homogen dispergiert.

Anschließend wurden die Suspensionen für 30 Minuten mit einer Kugelmühle (WAB, DYNO-MILL Research Lab, 70% Füllung mit 0,3-0,4mm Zirkoniabeads, 8m/s Tip Speed) in Kreisfahrweise vermahlen und anschließend gewogen. Anschließend wurden 22,5g Maltodextrin (GLUCIDEX 19) - korrigiert um die Ausbeute (Ausbeute in g/theoretische Ausbeute in g x 22,5g) - eingewogen und in der vermahlenen Suspension und Rühren gelöst. Die Suspensionen wurden zuletzt mit einem Laborsprühtrockner (Büchi B-290, 5ml/min Flow, Inlet Temperatur 145°C, 800l/h Sprühgas, 35 m3/h Aspirator) getrocknet.

Zur Referenz wurden ebenfalls native Cyclodextrin/Quercetin Komplexe (Nativ ACD, Nativ BCD, Nativ GCD) hergestellt, wobei zunächst nach demselben Verfahren vorgegangen wurde: 75g Cyclodextrin (CAVAMAX W6 bzw. W7 bzw. W8) sowie 20g Quercetin Dihydrat in 875ml destilliertem Wasser mit einem High Shear Homogenisator (IKA Ultra Turrax T 18) bei 5000 rpm dispergiert und anschließend mit wässriger Ammoniaklösung auf einen pH-Wert von 9 eingestellt. Zur Komplexbildung wurde die Suspension für weitere 30min bei 5000rpm dispergiert und der pH der Suspension anschließend durch Zugabe HCl-Lösung unter leichtem Rühren neutralisiert (finaler pH-Wert 2,5-3,5) und anschließend mit dem Sprühtrockner unter Anwendung derselben Sprühparameter getrocknet.

Zur Kontrolle wurden darüber hinaus Komplexe enthaltend Gamma-Cyclodextrin entweder nur 3% RS780 oder nur 3% Lipoid H100 als Additiv nach dem oben genannten Herstellverfahren produziert:
Komposition mit 3% RS780 (Q3B):

| **Bestandteil** | **Gewichtsanteil [Gew.-%]** |
|---|---|
| Quercetin Dihydrat | 16 |
| Gamma Cyclodextrin | 60 |
| Phosphatidylcholin /Lipoid H100 | 0 |
| Lycoat RS780 | 3 |
| Maltodextrin/Glucidex DE 19 | 21 |

Komposition mit 3% Lipoid H100 (Q3C):

| **Bestandteil** | **Gewichtsanteil [Gew.-%]** |
|---|---|
| Quercetin Dihydrat | 16 |
| Gamma Cyclodextrin | 60 |
| Phosphatidylcholin /Lipoid H100 | 3 |
| Lycoat RS780 | 0 |
| Maltodextrin/Glucidex DE 19 | 21 |

### 4.2 Sättigungslöslichkeit der Komplexe

Zur Messung der Sättigungslöslichkeit wurden Proben enthaltend 75mg Quercetin Dihydrat genau abgewogen und in 100ml biorelevantem Puffer (FASSIF-V2, Biorelevant: pH 6.5, Pufferkapazität 10mmol/delta pH/L enthaltend 3mM Natrium-Taurocholat; 0,2mM Lecithin; 19,12mM Maleinsäure 34,8mM; Natriumhydroxid; 68,62mM Natriumchlorid, entspricht bzgl. Zusammensetzung, pH-Wert, Ionenstärke sowie Pufferkapazität dem physiologischen Darmmedium) dispergiert und anschließend für 2h unter leichtem Schütteln inkubiert. Hiernach wurden die Proben mittels Spritzenfilter filtriert und die Quercetinkonzentration des Filtrats mittels HPLC bestimmt (berechnet als Dihydrat). Die Versuche wurden jeweils in Triplikaten angesetzt, die Durchschnittswerte sind tabellarisch dargestellt. Reines Quercetin Dihydrat wurde als Kontrolle mitgeführt.

| Komplex | CD | Verwendete Stärke | PC | Volumen FASSIF-V2 [ml] | Sättigungslöslic hkeit Quercetin in FASSIF-V2 [µg/ml] | Sättigungslöslic hkeit Quercetin in FASSIF-V2 [%] |
|---|---|---|---|---|---|---|
| Q1A | alpha | RS780 | Ja | 100 | 28,63 | 3,82 |
| Nativ ACD | alpha | keine | Nein | 100 | 23,45 | 3,13 |
| Q2A | beta | RS780 | Ja | 100 | 100,91 | 13,45 |
| Nativ BCD | beta | keine | Nein | 100 | 88,52 | 11,80 |
| Q3A | gamma | RS780 | Ja | 100 | 441,10 | 58,81 |
| Q3B | gamma | RS780 | Nein | 100 | 45,63 | 6,08 |
| Q3C | gamma | keine | Ja | 100 | 77,94 | 10,39 |
| Nativ GCD | gamma | keine | Nein | 100 | 40,66 | 5,42 |
| Quercetin | - | - | - | 100 | 14,40 | 1,92 |

Wenngleich der native Komplex enthaltend Beta-Cyclodextrin grundsätzlich höhere Löslichkeit in FASSIF-V2 besitzt, erhöht die erfinderische Formulierung lediglich bei gamma-Cyclodextrin signifikant die Löslichkeit im Vergleich zu dem nativen Komplex sowie absolut im Vergleich zu allen getesteten Formulierungen.

### 5. Wirkstoffformulierungen mit Boswelliasäuren

**Material:** Boswellia Serrata Extrakt (85% Boswelliasäuren, ermittelt durch Titration, Shaanxi Jia He Phytochem Co.,Ltd.), gamma-Cyclodextrin (CAVAMAX W8, Wacker Chemie), Phosphatidylcholin (96%, LIPOID H100, Lipoid), Maltodextrin (GLUCIDEX DE 19, Roquette), Lycoat RS780 (Roquette).

### 5.1 Komplexherstellung

Um den Einfluss des Gewichtsanteils der modifizierten Stärke sowie des Phosphatidylcholins zu ermitteln, wurden Komplexe mit den beiden Additiven Stärke (Lycoat RS780) und Phosphatidlycholin (Lipoid H100) sowie gamma-Cyclodextrin hergestellt, welche jedoch unterschiedliche Gewichtsanteile der beiden Additive enthielten.

### Komposition mit nur 1,5% RS780 (BS1A):

| **Bestandteil** | **Gewichtsanteil Gew.-%** |
|---|---|
| Boswellia Serrata Extrakt (85% Boswelliasäuren) | 27 |
| Gamma Cyclodextrin | 53 |
| Phosphatidylcholin /Lipoid H100 | 0 |
| Lycoat RS780 | 1,5 |
| Maltodextrin/Glucidex DE 19 | 18,5 |

### Komposition mit nur 1,5% Lipoid H100 (BS1B):

| **Bestandteil** | **Gewichtsanteil Gew.-%** |
|---|---|
| Boswellia Serrata Extrakt (85% Boswelliasäuren) | 27 |
| Gamma Cyclodextrin | 53 |
| Phosphatidylcholin /Lipoid H100 | 1,5 |
| Lycoat RS780 | 0 |
| Maltodextrin/Glucidex DE 19 | 18,5 |

### Komposition mit jeweils 0,5% RS780 und Lipoid H100 (BS2A):

| **Bestandteil** | **Gewichtsanteil Gew.-%** |
|---|---|
| Boswellia Serrata Extrakt (85% Boswelliasäuren) | 27 |
| Gamma Cyclodextrin | 53 |
| Phosphatidylcholin /Lipoid H100 | 0,5 |
| Lycoat RS780 | 0,5 |
| Maltodextrin/Glucidex DE 19 | 19 |

### Komposition mit jeweils 1,5% RS780 und Lipoid H100 (BS2B):

| **Bestandteil** | **Gewichtsanteil Gew.-%** |
|---|---|
| Boswellia Serrata Extrakt (85% Boswelliasäuren) | 27 |
| Gamma Cyclodextrin | 53 |
| Phosphatidylcholin /Lipoid H100 | 1,5 |
| Lycoat RS780 | 1,5 |
| Maltodextrin/Glucidex DE 19 | 17 |

### Komposition mit jeweils 3% RS780 und Lipoid H100 (BS2C):

| **Bestandteil** | **Gewichtsanteil Gew.-%** |
|---|---|
| Boswellia Serrata Extrakt (85% Boswelliasäuren) | 27 |
| Gamma Cyclodextrin | 53 |
| Phosphatidylcholin /Lipoid H100 | 3 |
| Lycoat RS780 | 3 |
| Maltodextrin/Glucidex DE 19 | 14 |

### Komposition mit 1,5% RS780 und 3% Lipoid H100 (BS3A):

| **Bestandteil** | **Gewichtsanteil Gew.-%** |
|---|---|
| Boswellia Serrata Extrakt (85% Boswelliasäuren) | 27 |
| Gamma Cyclodextrin | 53 |
| Phosphatidylcholin /Lipoid H100 | 3 |
| Lycoat RS780 | 1,5 |
| Maltodextrin/Glucidex DE 19 | 15,5 |

### Komposition mit 3% RS780 und 1,5% Lipoid H100 (BS3B):

| **Bestandteil** | **Gewichtsanteil Gew.-%** |
|---|---|
| Boswellia Serrata Extrakt (85% Boswelliasäuren) | 27 |
| Gamma Cyclodextrin | 53 |
| Phosphatidylcholin /Lipoid H100 | 1,5 |
| Lycoat RS780 | 3 |
| Maltodextrin/Glucidex DE 19 | 15,5 |

Hierzu wurden zunächst 79,5g gamma-Cyclodextrin (CAVAMAX W8) sowie 40,5g Boswellia Serrata Extrakt in 850ml destilliertem Wasser mit einem High Shear Homogenisator (IKA Ultra Turrax T 18) bei 6000 rpm für 1-2 Minuten eingerührt und anschließend zur Komplexbildung für weitere 30min bei 4000rpm dispergiert. Hiernach wurde die jeweils berechnete Menge - je nach Komposition des Komplexes - an modifizierter Stärke RS780) sowie Phosphatidylcholin (Lipoid H100) unter Rühren homogen dispergiert.

Anschließend wurde die Lösung mittels Hochdruckhomogenisator (GEA, PandaPlus 1000) bei 400 bar in 3 Zyklen homogenisiert und anschließend gewogen.

Anschließend wurde die je nach Komposition des Komplexes benötigte Menge Maltodextrin (GLUCIDEX 19) - korrigiert um die Ausbeute - eingewogen und in der vermahlenen Suspension und Rühren gelöst.

Die Suspensionen wurden zuletzt mit einem Laborsprühtrockner (Büchi B-290, 5ml/min Flow, Inlet Temperatur 140°C, 600l/h Sprühgas, 35 m3/h Aspirator) getrocknet.

Zur Referenz wurde ebenfalls ein nativer Gamma-Cyclodextrin/Boswellia Komplex (Nativ GCD) hergestellt, wobei zunächst nach demselben Verfahren vorgegangen wurde: 79,5g gamma-Cyclodextrin (CAVAMAX W8) sowie 40,5g Boswellia Serrata Extrakt wurden in 850ml destilliertem Wasser mit einem High Shear Homogenisator (IKA Ultra Turrax T 18) bei 6000 rpm für 1-2 Minuten eingerührt und anschließend zur Komplexbildung für weitere 30min bei 4000rpm dispergiert. Anschließend erfolgte die Trocknung mittels Sprühtrockner unter Anwendung der oben genannten Sprühparameter.

### 5.2 in-vitro Freisetzung der Formulierungen

Anschließend wurde die in-vitro Freisetzung nach Vorgaben des Arzneibuches (Ph. Eur. 11.0 Kapitel 2.9.3. Freisetzung fester Darreichungsformen) untersucht. Hierzu wurde der USP Apparatus II (Paddle/Blattrührer) ausgewählt, die Umdrehungsgeschwindigkeit betrug 50rpm, V=500ml Fassif-V2 Medium, T= 37°C.

Der Gehalt an Acetyl-11-keto-β-boswelliasäure (AKBA) sowie 11-Keto-β-Boswelliasäure (KBA) wurde für jeden der hergestellten Komplexe bestimmt, diese beiden Boswelliasäuren werden für die Freisetzungsversuche als Analyten herangezogen.

Es wurden Proben enthaltend 12,5mg Boswelliasäuren (AKBA + KBA) genau eingewogen, entsprechend einer Zielkonzentration von 25µg/ml AKBA+KBA. Nachdem das Medium auf Zieltemperatur gebracht und die Proben in die Gefäße gegeben wurden, erfolgte der Probenzug nach 15, 30, 60 und 120 Minuten, wobei die Proben mittels Spritzenfilter filtriert und die Boswelliasäurekonzentration mittels HPLC bestimmt wurde. Entnommenes Probenvolumen wurde sofort nach Entnahme durch frisches, vortemperiertes FASSIF-V2 Medium ersetzt. Die Versuche wurden jeweils in Triplikaten angesetzt, die Durchschnittswerte der Freisetzung (Q in %) sind tabellarisch dargestellt.

| Komplex | Anteil RS780 [%] | Anteil PC [%] | Q nach 15min [%] | Q nach 30min [%] | Q nach 60min [%] | Q nach 120min [%] |
|---|---|---|---|---|---|---|
| BS1A | 1,5 | 0 | 11,72 | 13,41 | 16,89 | 16,42 |
| BS1B | 0 | 1,5 | 15,94 | 16,38 | 17,02 | 17,84 |
| BS2A | 0,5 | 0,5 | 24,86 | 28,28 | 26,95 | 23,43 |
| BS2B | 1,5 | 1,5 | 33,51 | 36,89 | 38,76 | 39,44 |
| BS2C | 3 | 3 | 21,57 | 26,93 | 27,45 | 28,82 |
| BS3A | 1,5 | 3 | 41,42 | 45,89 | 47,32 | 46,93 |
| BS3B | 3 | 1,5 | 24,27 | 23,44 | 23,78 | 25,96 |
| Nativ GCD | keine | keine | 9,03 | 12,42 | 14,86 | 16,80 |
| Boswellia Serrata Extrakt | - | - | 0,61 | 0,93 | 1,47 | 2,01 |

### 6. Wirkstoffformulierungen mit Berberin

**Material:** Berberin HCl (97,9% Thermo Fisher Scientific), gamma-Cyclodextrin (CAVAMAX W8, Wacker Chemie), Phosphatidylcholin (96%, LIPOID H100, Lipoid), Maltodextrin (GLUCIDEX DE 19, Roquette), Lycoat RS780 (Roquette).

### 6.1 Komplexherstellung

### Es wurde folgender Komplex enthaltend Berberin HCl hergestellt (BB1A):

| **Bestandteil** | **Gewichtsanteil Gew.-%** |
|---|---|
| Berberin HCl | 18 |
| Gamma Cyclodextrin | 62 |
| Phosphatidylcholin /Lipoid H100 | 1,75 |
| Lycoat RS780 | 1,75 |
| Maltodextrin/Glucidex DE 19 | 16,5 |

Hierzu wurden zunächst 62g gamma-Cyclodextrin (CAVAMAX W8) sowie 18g Berberin HCl in 900ml destilliertem Wasser mit einem High Shear Homogenisator (IKA Ultra Turrax T 18) bei 10.000 rpm für 1-2 Minuten eingerührt und anschließend zur Komplexbildung für weitere 30min bei 8000rpm dispergiert. Hiernach wurden jeweils 1,75g modifizierter Stärke (RS780) sowie Phosphatidylcholin (Lipoid H100) unter Rühren homogen dispergiert.

Anschließend wurde die Lösung mittels Hochdruckhomogenisator (GEA, PandaPlus 1000) bei 500 bar in 1 Zyklus homogenisiert und anschließend gewogen.

Anschließend wurden 16 Maltodextrin (GLUCIDEX 19) - korrigiert um die Ausbeute - eingewogen und in der vermahlenen Suspension und Rühren gelöst.

Die Suspensionen wurden zuletzt mit einem Laborsprühtrockner (Büchi B-290, 5ml/min Flow, Inlet Temperatur 140°C, 800l/h Sprühgas, 35 m3/h Aspirator) getrocknet.

Zur Referenz wurde ebenfalls ein nativer Gamma-Cyclodextrin/Berberin Komplex (Nativ GCD) hergestellt, wobei zunächst nach demselben Verfahren vorgegangen wurde: 62g gamma-Cyclodextrin (CAVAMAX W8) sowie 18g berberin HCl wurden in 900ml destilliertem Wasser mit einem High Shear Homogenisator (IKA Ultra Turrax T 18) bei 10.000 rpm für 1-2 Minuten eingerührt und anschließend zur Komplexbildung für weitere 30min bei 8000rpm dispergiert. Anschließend erfolgte die Trocknung mittels Sprühtrockner unter Anwendung der oben genannten Sprühparameter.

### 6.2 Sättigungslöslichkeit der Komplexe

Zur Messung der Sättigungslöslichkeit wurden Proben enthaltend 20mg Berberin HCl genau abgewogen und in 2ml 0,05M Phosphatpuffer pH 6.8 dispergiert und anschließend für 2h unter leichtem Schütteln inkubiert. Hiernach wurden die Proben mittels Spritzenfilter filtriert und die Berberinkonzentration des Filtrats mittels HPLC bestimmt. Die Versuche wurden jeweils in Triplikaten angesetzt, die Durchschnittswerte sind tabellarisch dargestellt.

Die Versuche wurden mit BB1A, dem nativen Berberin/gamma-Cyclodextrin Komplex sowie reinem Berberin HCl durchgeführt.

| Komplex | CD | Additive | Volumen PBS pH 6.8 [ml] | Sättigungslöslichkeit Berberin in PBS pH 6.8 [mg/ml] | Sättigungslöslichkeit Berberin in PBS pH 6.8 [%] |
|---|---|---|---|---|---|
| BB1A | gamma | 1,75% RS780 + 1,75% H100 | 2 | 8,69 | 86,9 |
| Nativ GCD | gamma | keine | 2 | 2,86 | 28,6 |
| Berberin HCl | - | - | 2 | 1,38 | 13,8 |

Es konnte durch die erfinderische Formulierung eine signifikante Erhöhung der Sättigungslöslichkeit im Vergleich zu dem reinen Wirkstoff sowie dem gamma-Cyclodextrin Komplex erzielt werden.

## Patentansprüche

1. Zusammensetzung, umfassend mindestens einen Cyclodextrin-Komplex, worin mindestens ein Wirkstoff mit gamma-Cyclodextrin assoziiert ist, wobei der Cyclodextrin-Komplex eine Beschichtung aufweist, die Hydroxypropylstärke und Phosphatidylcholin umfasst,
wobei der Gewichtsanteil des Wirkstoffs 5-50 Gew.-% beträgt, bevorzugt 10-40 Gew.-%,
der Gewichtsanteil des gamma-Cyclodextrins 35-90 Gew.-% beträgt, bevorzugt 50-85 Gew.-%,
der Gewichtsanteil der Hydroxypropylstärke 0,1-10 Gew.-% beträgt, bevorzugt 0,5-7,55 Gew.-%, weiter bevorzugt 0,75-4 Gew.-%, besonders bevorzugt 1-3,5 Gew.-%, und der Gewichtsanteil des Phosphatidylcholins 0,1-10 Gew.-% beträgt, bevorzugt 0,5-7,5 Gew.-% beträgt, weiter bevorzugt 1-4,5 Gew.-%, besonders bevorzugt 1,5-4 Gew.-%, jeweils bezogen auf die Gesamtmasse des beschichteten Komplexes.

2. Zusammensetzung nach Anspruch 1, wobei der Wirkstoff ein Molekulargewicht von 150-2000 g/mol und/oder eine Wasserlöslichkeit von < 10 mg/ml aufweist, bevorzugt < 1 mg/ml aufweist.

3. Zusammensetzung nach Anspruch 1 oder 2, wobei die Zusammensetzung in fester Form vorliegt, insbesondere als Pulver oder Granulat.

4. Zusammensetzung nach einem der vorhergehenden Ansprüche, wobei die Hydroxypropylstärke eine Viskosität von < 250mPas aufweist, bevorzugt < 100mPas, besonders bevorzugt < 75 mPas, am meisten bevorzugt < 50mPas, wie mittels Rotationsviskosimetrie bestimmt, und/oder wobei die Hydroxypropylstärke einen Substitutionsgrad von 0,1-15 % aufweist, bevorzugt 0,5-10 %, weiter bevorzugt 2-7 %, wie mittels Kernspinresonanzspektroskopie bestimmt.

5. Zusammensetzung nach einem der vorhergehenden Ansprüche, wobei der Gesamtanteil des Wirkstoffs und des gamma-Cyclodextrins zusammen 85-99 Gew.-% beträgt, bevorzugt 90-98,5 Gew.-%, besonders bevorzugt 93-98 Gew.-%, bezogen auf die Gesamtmasse des beschichteten Komplexes.

6. Zusammensetzung nach einem der vorhergehenden Ansprüche, wobei der Gesamtanteil des Phosphatidylcholins und der Hydroxypropylstärke zusammen 0,5-15 Gew.-% beträgt, bevorzugt 1-10 Gew.-%, weiter bevorzugt 1,5-8,5 Gew.-%, besonders bevorzugt 2-7 Gew.-%, bezogen auf die Gesamtmasse des beschichteten Komplexes.

7. Zusammensetzung nach einem der vorhergehenden Ansprüche, wobei die Zusammensetzung weiterhin ein oder mehrere Saccharide umfasst, vorzugsweise in einer Menge von 5-50 Gew.-%, bevorzugt 10-40 Gew.-%, besonders bevorzugt 12,5-35 Gew.-%, am meisten bevorzugt 15-30 Gew.-%, bezogen auf die Gesamtmasse des beschichteten Komplexes,
optional wobei die ein oder mehreren Saccharide aus der Gruppe ausgewählt sind, die Maltodextrin, Glucose, Saccharose, Fructose, Maltose, Lactose, Galactose, Trehalose, Mannose und Kombinationen davon umfasst.

8. Zusammensetzung nach einem der vorhergehenden Ansprüche, wobei die beschichteten Cyclodextrin-Komplexe in Form von Agglomeraten mehrerer beschichteter Cyclodextrin-Komplexe vorliegen.

9. Zusammensetzung nach einem der vorhergehenden Ansprüche, wobei die Zusammensetzung eine Zusammensetzung für die orale Verabreichung ist, insbesondere eine Tablette, Kapsel, Kau-, Schmelz- oder Brausetablette, ein Granulat, Pulver, eine Lösung für die orale Einnahme, ein Dragee, eine Sublingual/Buccaltablette oder ein Schmelzfilm.

10. Zusammensetzung nach einem der vorhergehenden Ansprüche, wobei die Zusammensetzung eine pharmazeutische oder nutrazeutische Zusammensetzung oder ein Lebensmittel ist.

11. Verfahren zur Herstellung einer Zusammensetzung nach einem der Ansprüche 1-10, umfassend die Schritte:
a) Dispergieren von gamma-Cyclodextrin und mindestens einem Wirkstoff in Wasser unter Rühren, um den Wirkstoff mit dem gamma-Cyclodextrin zu assoziieren,
b) Beschichten des in Schritt (a) gebildeten Cyclodextrin-Komplexes durch Zugeben von Hydroxypropylstärke und Phosphatidylcholin unter Rühren und Homogenisieren, um eine Suspension beschichteter Cyclodextrin-Komplexe in Wasser zu erhalten, und
c) ggf. Trocknen der in Schritt (b) erhaltenen Suspension, insbesondere durch Sprühtrocknen.

12. Verfahren nach Anspruch 11, wobei Schritt (a) weiterhin das Einstellen des pH-Werts umfasst, um die Komplexierung des Wirkstoffes mit gamma-Cyclodextrin zu fördern.

13. Verfahren nach Anspruch 11 oder 12, wobei die Homogenisierung in Schritt (b) mittels Hochdruck-Homogenisation, Ultraschall-Homogenisation oder Vermahlen, beispielsweise Kugelmahlen, erfolgt.

14. Verfahren nach einem der Ansprüche 11-13, wobei der Suspension beschichteter Cyclodextrin-Komplexe nach Schritt (b) weiterhin ein oder mehrere Saccharide zugegeben werden.

15. Verfahren nach einem der Ansprüche 11-14, wobei Phosphatidylcholin in Form einer Phospholipidmischung zugegeben wird, die mindestens 40 Gew.-%, vorzugsweise mindestens 80 Gew.-%, weiter bevorzugt mindestens 90 Gew.-% Phosphatidylcholin umfasst.
